(19) Europäisches Patentamt
European Patent Office
Office européen des brevets

(11) EP 1 172 093 B1

(12) **FASCICULE DE BREVET EUROPEEN**

(45) Date de publication et mention de la délivrance du brevet:
**01.12.2004 Bulletin 2004/49**

(51) Int Cl.[7]: **A61K 7/48**

(21) Numéro de dépôt: **01401748.7**

(22) Date de dépôt: **29.06.2001**

(54) **Composition contenant des composés du type sel de flavylium non substitués en position 3 pour la coloration de la peau et une silicone organomodifiée**

Hautfärbemittel enthaltend Flavylium-Salz Verbindungen, die in Position 3 unsubstituierte sind, und eine organomodifizierte Silikon

Compositions for coloring the skin containing flavylium salt compounds unsubstituted in position 3 and an organomodified silicone

(84) Etats contractants désignés:
**AT BE CH CY DE DK ES FI FR GB GR IE IT LI LU MC NL PT SE TR**

(30) Priorité: **12.07.2000 FR 0009116**

(43) Date de publication de la demande:
**16.01.2002 Bulletin 2002/03**

(73) Titulaire: **L'OREAL**
**75008 Paris (FR)**

(72) Inventeurs:
- **Candau, Didier**
  **91570 Bievres (FR)**
- **Forestier, Serge**
  **77410 Claye-Souilly (FR)**
- **Elguidj, Irène**
  **92200 Neuilly (FR)**

(74) Mandataire: **Miszputen, Laurent**
**L'OREAL - D.I.P.I.**
**25-29 Quai Aulagnier**
**92600 Asnières (FR)**

(56) Documents cités:
**FR-A- 2 757 383**

- **La recherche additionnelle effectuée n'a pas permis de révéler de documents supplémentaires.**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 172 093 B1

## Description

La présente invention a pour objet une émulsion cosmétique ou dermatologique, caractérisée par le fait qu'elle comporte :

- au moins une phase aqueuse et
- au moins une phase grasse ;
- au moins un composé du type sel de flavylium, non substitué en position 3, et substitué par au moins un radical hydroxyle ou alcoxy, obtenu par voie de synthèse ou à partir d'un extrait végétal le contenant ou bien encore à partir d'un extrait végétal enrichi, dans une quantité efficace permettant d'obtenir au bout de 30 minutes après application sur une peau claire à raison de $2mg/cm^2$ un assombrissement de la couleur de peau caractérisé dans le système de mesure colorimétrique L* a* b* par un ΛL* allant de -0,5 à -20.
- au moins une silicone organomodifiée.

De nos jours, il est important d'avoir bonne mine et une peau bronzée est toujours signe de bonne santé. Cependant, le bronzage naturel n'est pas toujours souhaitable dans la mesure où il nécessite des expositions prolongées aux rayonnements UV, en particulier aux rayonnements UV-A qui provoquent le brunissement de la peau mais en contrepartie sont susceptibles d'induire une altération de celle-ci, notamment dans le cas d'une peau sensible ou d'une peau continuellement exposée au rayonnement solaire. Il est donc souhaitable de trouver une alternative au bronzage naturel qui soit compatible avec les exigences de telles peaux.

La plupart des produits cosmétiques destinés au bronzage artificiel de la peau sont à base de dérivés carbonylés permettant, par interaction avec les acides aminés de la peau, la formation de produits colorés.

A cet effet, on sait que la dihydroxyacétone, ou DHA, est un produit particulièrement intéressant qui est couramment utilisé en cosmétique comme agent de bronzage artificiel de la peau; appliqué sur cette dernière, notamment sur le visage, il permet d'obtenir un effet de bronzage ou de brunissage d'apparence semblable à celui qui peut résulter d'une exposition prolongée au soleil (bronzage naturel) ou sous une lampe UV.

Un inconvénient de la DHA est la lenteur avec laquelle la coloration se développe : il faut en effet compter plusieurs heures (3 à 5 heures en général) pour que soit révélée la coloration. Il existe donc une demande croissante de produits autobronzants agissant rapidement et conférant une coloration plus proche du bronzage naturel.

Ainsi, on est toujours à la recherche de nouveaux composés et de nouvelles compositions permettant de conférer artificiellement à la peau une coloration proche du bronzage naturel d'une manière simple, efficace, rapide et sans risque.

Les colorants anthocyaniques sont connus depuis longtemps comme colorants alimentaires et pharmaceutiques. Ces anthocyanes sont présents dans la nature sous forme d'hétérosides appelés anthocyanosides et de génines, appelées anthocyanidines. Ces anthocyanes sont des dérivés du phényl-2-benzopyrylium ou flavylium et sont notamment présents dans la plante sous forme de sels. Les anthocyanes sont des composés de teinte rouge, violette ou bleue qui colorent généralement les fleurs, les fruits et parfois les feuilles. La couleur observée dépend à la fois de la structure de la génine majoritaire et des conditions du milieu où se trouvent les colorants anthocyaniques.

Or, à la suite d'importantes recherches menées dans le domaine de la coloration artificielle de la peau, la Demanderesse a maintenant découvert que des émulsions particulières contenant à titre d'agent de coloration de la peau au moins un composé particulier du type sel de flavylium non substitué en position 3 et substitué par au moins un radical hydroxyle ou alcoxy et au moins une silicone organomodifiée, permettaient non seulement de conférer immédiatement après l'application sur la peau du produit une coloration artificielle proche du bronzage naturel mais aussi présentaient une bonne stabilité ainsi qu'un bon agrément cosmétique.

Ces découvertes sont à l'origine de la présente invention.

La présente invention a donc pour objet une émulsion cosmétique ou dermatologique, caractérisée par le fait qu'elle comporte :

(a) au moins une phase aqueuse et
(b) au moins une phase grasse ;
(c) au moins un composé particulier du type sel de flavylium non substitué en position 3 et substitué par au moins un radical hydroxyle ou alcoxy, dans une quantité efficace permettant d'obtenir au bout de 30 minutes après application sur une peau claire à raison de $2mg/cm^2$ un assombrissement de la couleur de peau caractérisé dans le système de mesure colorimétrique L* a* b* par un ΔL* allant de -0,5 à -20 ;
(d) au moins une silicone organomodifiée.

Par émulsion cosmétique ou dermatologique, au sens de la présente invention et dans le texte qui suit, on entend toute émulsion dont la phase aqueuse et la phase grasse sont constituées de substances cosmétiquement ou

dermatologiquement acceptables pour une application topique sur la peau.

La présente invention a encore pour objet l'utilisation nouvelle d'au moins un composé du type sel de flavylium, non substitué en position 3, et substitué par au moins un radical hydroxyle ou alcoxy, obtenu par voie de synthèse, ou à partir d'un extrait végétal, ou d'un extrait végétal enrichi le contenant, et d'au moins une silicone organomodifiée dans des compositions cosmétiques et/ou dermatologiques dans le but de conférer à la peau une coloration artificielle proche du bronzage naturel.

La présente invention a également pour objet un procédé de coloration artificielle proche du bronzage naturel de la peau, caractérisé en ce qu'il consiste à appliquer sur celle-ci une quantité efficace d'au moins un composé du type sel de flavylium, non substitué en position 3, et substitué par au moins un radical hydroxyle ou alcoxy, obtenu par voie de synthèse, ou à partir d'un extrait végétal, ou d'un extrait végétal enrichi et d'au moins une silicone organomodifiée, dans une composition cosmétique le contenant.

La présente invention a également pour objet l'utilisation de l'émulsion pour la fabrication de compositions cosmétiques pour la coloration de la peau.

La présente invention a également pour objet l'utilisation d'une silicone organomodifiée pour la fabrication d'une émulsion cosmétique ou dermatologique contenant au moins au moins un composé du type sel de flavylium, non substitué en position 3, et substitué par au moins un radical hydroxyle ou alcoxy dans ladite émulsion, dans le but d'obtenir une coloration de la peau immédiate tout en ayant une bonne stabilité physique et un bon agrément cosmétique.

Les compositions et les utilisations conformes à l'invention permettent d'obtenir une coloration artificielle proche du bronzage naturel en un laps de temps court. Ainsi, on obtient une coloration immédiate qui permet une visualisation de l'application et par conséquent une meilleure homogénéité dans l'étalement de la composition sur la peau et donc de la coloration qui en résulte.

Au sens de la présente invention, on entendra, par « composition destinée à la coloration artificielle de la peau », une formulation ayant une affinité particulière pour la peau lui permettant de conférer à cette dernière une coloration durable, non-couvrante (à savoir n'ayant pas tendance à opacifier la peau) et qui ne s'élimine ni à l'eau ni à l'aide d'un solvant, et qui résiste à la fois au frottement et au lavage par une solution contenant des tensioactifs. Une telle coloration durable se distingue donc de la coloration superficielle et momentanée apportée par exemple par un produit de maquillage.

On entend par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en $C_1$-$C_{10}$ et en particulier méthyle, les radicaux fluoroalkyles, les radicaux aryles et en particulier phényle. Les silicones sont définies plus en détail dans l'ouvrage de Walter NOLL "Chemistry and Technology of Silicones" (1968) Academie Press.

D'autres caractéristiques, aspects et avantages de la présente invention apparaîtront à la lecture de la description détaillée qui va suivre.

Les silicones organomodifiées utilisables conformément à l'invention sont des silicones telles que définies précédemment et comportant dans leur structure un ou plusieurs groupements organofonctionnels fixés par l'intermédiaire d'un radical hydrocarboné. Les silicones utilisables conformément à invention peuvent se présenter sous forme d'huiles, de cires, de résines ou de gommes. Elles peuvent être hydrosolubles ou insolubles dans l'eau.

Parmi les silicones organomodifiées, on peut citer les polyorganosiloxanes comportant

(1) des groupements oxyalkylénés (en particulier oxyéthylénés et/ou oxypropylénés) telles que celles décrites dans la demande de brevet EP-0796615 et répondant aux formules suivantes :

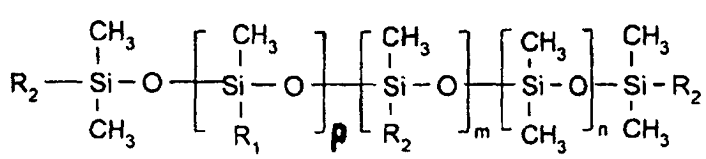 (I)

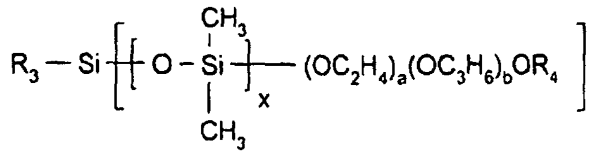 (II)

- $R_1$, identique ou différent, représente un radical alkyle, linéaire ou ramifié, en $C_1$-$C_{30}$ ou phényle.
- $R_2$, identique ou différent, $-C_cH_{2c}-(-O-C_2H_4)_a-(-O-C_3H_6)_b-(O-C_4H_8)_d-R_5$
- $R_3$, $R_4$, identiques ou différents, désignent un radical alkyle, linéaire ou ramifié, en $C_1$-$C_{12}$, et de préférence le radical méthyle,
- $R_5$, identique ou différent, est choisi parmi un atome d'hydrogène, un radical hydroxyle, un radical alkyle linéaire ou ramifié de 1 à 12 atomes de carbone, un radical alcoxy linéaire ou ramifié de 1 à 6 atomes de carbone, un radical acyloxy linéaire ou ramifié de 2 à 12 atomes de carbone, $NHCH_2CH_2COOM$, aminoalkyle éventuellement substitué sur l'amine, carboxyacyle en $C_1$-$C_{30}$, un groupement phosphono éventuellement substitué, -O-CO-$(CH_2)_d$-$CO_2M$, -NHCO$(CH_2)_d$OH, -$NH_3$Y.
- M, identique ou différent, désigne un atome d'hydrogène, Na, K, Li, $NH_4$ ou une amine organique,
- a varie de 0 à 50,
- b varie de 0 à 50,
- c varie de 0 à 4,
- a + b est supérieur ou égal à 1,
- d varie de 0 à 10,
- m varie de 0 à 20,
- n varie de 0 à 500,
- p varie de 0 à 20,
- x varie de 1 à 100,
- Y représente un anion minéral ou organique monovalent tel que halogénure (chlorure, bromure), sulfate, carboxylate (acétate, lactate, citrate) ; comme par exemple celles vendues sous les dénominations commerciales FLUID DC 193 par la société DOW CORNING, SILWET L 77 par la société OSI et MAZIL 756 par la société MAZER PPG ; on peut citer également les produits commercialisés sous les noms « Silicone DC 3225C » « DC Q2-5200 » par la société Dow Corning ;

(2) des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;

(3) des groupements anioniques du type 2-hydroxyalkylsulfonate ; 2-hydroxyalkylthiosulfate tels que les produits commercialisés par la société GOLDSCHMIDT sous les dénominations "ABIL S201" et "ABIL S255" ;

(4) des groupements thiols comme les produits commercialisés sous les dénominations "GP 72 A" et "GP 71" de GENESEE ;

(5) des groupements anioniques du type carboxylique telles que les silicones organomodifiées décrites dans les demande WO95/23579 et EP-A-0 219 830, WO98/20883 et notamment celles répondant à la formule (III) suivante :

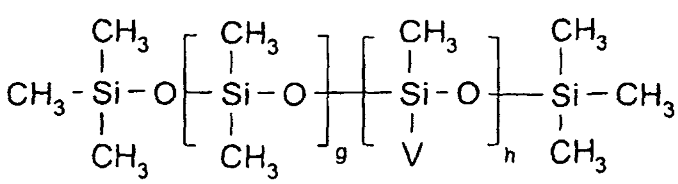 (III)

dans laquelle V est un radical $-(R^1O)_e-R^2-(OR^3)_f-COOM$, dans lequel $R^1$ et $R^3$ désignent indépendamment un radical alkylène linéaire ou ramifié ayant de 2 à 20 atomes de carbone, $R^2$ désigne un radical alkylène linéaire ou ramifié ayant de 1 à 50 atomes de carbone pouvant comprendre un groupement hydroxyle ; e représente 0 ou 1 et f est un nombre allant de 0 à 200 ; M désigne hydrogène, un métal alcalin ou alcalino-terreux, $NH_4$, un groupe-

ment ammonium quaternaire tel qu'un groupement mono-, di-, tri- ou tétra (alkyl $C_1$-$C_4$) ammonium ; g et h sont des nombres allant de 0 à 1000, la somme c+d allant de préférence de 2 à 1000 ;par exemple celles commercialisés sous la dénomination Huile M 642 par la société WACKER, sous les dénominations SLM 23 000/1, SLM 23 000/2 par la société WACKER , sous la dénomination 176-12057 par la société GENERAL ELECTRIC, sous la dénomination FZ 3703 par la société OSI, sous la dénomination BY 16 880 par la société TORAY SILICONE ; on peut citer également les produits décrits dans le brevet EP 186 507 de la société CHISSO CORPORATION, ou de type alkylcarboxyliques comme ceux présents dans le produit X-22-3701E de la société SHIN-ETSU ;

(6) des groupements hydroxylés comme les polyorganosiloxanes à fonction hydroxyalkyle décrits dans la demande de brevet français FR-A-85 16334 répondant à la formule (IV) :

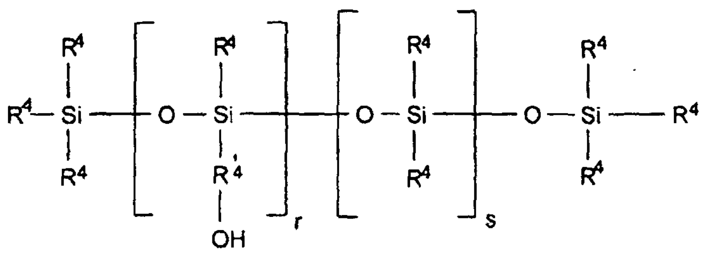

(IV)

dans laquelle les radicaux $R^4$ identiques ou différents sont choisis parmi les radicaux méthyle et phényle ; au moins 60 % en mole des radicaux $R^4$ désignant méthyle ; le radical $R'^4$ est un chaînon alkylène divalent hydrocarboné en $C_2$-$C_{18}$ ; r est compris entre 1 et 30 inclus ; s est compris entre 1 et 150 inclus ;

(7) des groupements acyloxyalkyle tels que par exemple les polyorganosiloxanes décrits dans la demande de brevet français FR-A-2641185 et répondant à la formule (V) :

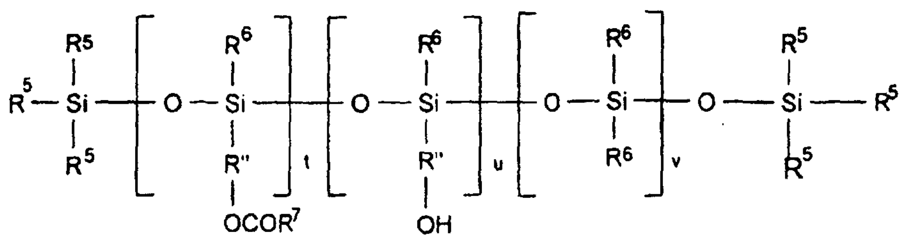

(V)

dans laquelle:

$R^5$ désigne un groupement méthyle, phényle, $-OCOR_5$, hydroxyle, un seul des radicaux $R^5$ par atome de silicium pouvant être OH ;
$R^6$ désigne méthyle, phényle ; au moins 60 % en proportion molaire de l'ensemble des radicaux $R^5$, et $R^6$, désignant méthyle;
$R^7$ désigne alkyle ou alcényle en $C_8$-$C_{20}$ ;
R" désigne un radical alkylène hydrocarboné divalent, linéaire ou ramifié en $C_2$-$C_{18}$ ;
t est compris entre 1 et 120 inclus ;
u est compris entre 1 et 30 ;
v est égal à 0 ou est inférieur à 0,5 t, t + u étant compris entre 1 et 30 ; les polyorganosiloxanes de formule (V) peuvent contenir des groupements :

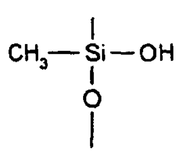

dans des proportions ne dépassant pas 15 % de la somme t + u + v ;

(8) des groupements aminés substitués ou non telles que les silicones décrites dans la demande de brevet EP-A-0852 488 et notamment les silicones aminées choisies parmi :

(a) les polysiloxanes répondant à la formule :

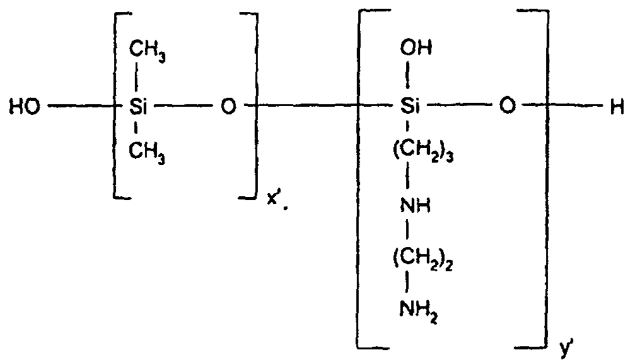

(VI)

dans laquelle x' et y' sont des nombres entiers dépendant du poids moléculaire, généralement tels que ledit poids moléculaire en nombre est compris entre 5 000 et 500 000 ;

(b) les polymères cationiques siliconés répondant à la formule:

$$R^8{}_iG_{3-i}\text{-}Si(OSiG_2)_k\text{-}(OSiG_lR^8{}_{2-j})_l\text{-}O\text{-}SiG_{3-i}\text{-}R^8{}_i \qquad \text{(VII)}$$

dans laquelle:

G est un atome d'hydrogène, ou un groupement phényle, OH, ou alkyle en $C_1$-$C_8$, par exemple méthyle,
i désigne le nombre 0 ou un nombre entier de 1 à 3, en particulier 0,
j désigne 0 ou 1, et en particulier 1,
k et l sont des nombres tels que la somme (k+l) peut varier notamment de 1 à 2 000 et en particulier de 50 à 150, k pouvant désigner un nombre de 0 à 1 999 et notamment de 49 à 149 et k prouvant désigner un nombre de 1 à 2 000, et notamment de 1 à 10 ;
$R^8$ est un radical monovalent de formule $-C_qH_{2q}L$ dans laquelle q est un nombre de 2 à 8 et L est un groupement aminé éventuellement quaternisé choisi parmi les groupements :

$$-NR^9\text{-}CH_2\text{-}CH_2\text{-}N(R^9)_2$$

$$-N(R^9)_2$$

$$-N^{\oplus}(R^9)_3A^-$$

$$-N(R^9)\text{-}CH_2\text{-}CH_2\text{-}N^{\oplus}R^9H_2A^-,$$

dans lesquels $R^9$ désigne hydrogène, phényle, benzyle, ou un radical hydrocarboné saturé monovalent, par exemple un radical alkyle ayant de 1 à 20 atomes de carbone et $A^-$ représente un ion halogénure tel que par exemple fluorure, chlorure, bromure ou iodure.

(c) les polymères cationiques siliconés répondant à la formule :

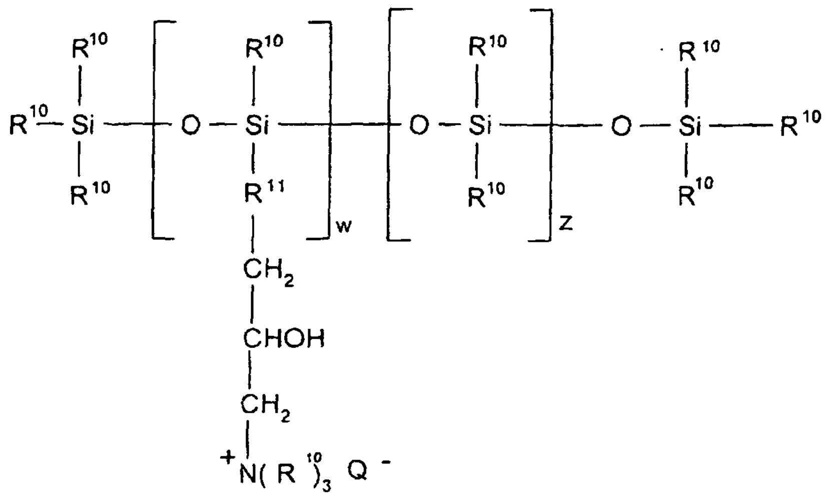

(VIII)

dans laquelle :

$R^{10}$ représente un radical hydrocarboné monovalent ayant de 1 à 18 atomes de carbone, et en particulier un radical alkyle en $C_1$-$C_{18}$, ou alcényle en $C_2$-$C_{18}$, par exemple méthyle ;
$R^{11}$ représente un radical hydrocarboné divalent, notamment un radical alkylène en $C_1$-$C_{18}$ ou un radical alkylèneoxy divalent en $C_1$-$C_{18}$, par exemple en $C_1$-$C_8$ ;
$Q^-$ est un ion halogénure, notamment chlorure ;
w représente une valeur statistique moyenne de 2 à 20 et en particulier de 2 à 8 ;
z représente une valeur statistique moyenne de 20 à 200 et en particulier de 20 à 50 ; à titre d'exemple de silicones aminées, on peut citer les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING.;

(9) des groupements hydroxyacylamino, comme les polyorganosiloxanes décrits dans la demande EP 342 834. On peut citer par exemple le produit Q2-8413 de la société DOW CORNING.

(10) des groupements de type aryle éventuellement substitué comme par exemple phényle, naphtyle, benzyle ou phénéthyle telles que les silicones arylées non-volatiles décrites dans la demande de brevet EP-A-822202 ; à titre d'exemples de ces composés, on peut citer ceux vendus par la société BAYER sous le nom Huile BAYSILONE FLUID PD5, par la société DOW CORNING sous le nom DOW CORNING 556 FLUID, par RHONE POULENC sous les noms MIRASIL DPDM, RHODORSIL HUILE 510 V 100, RHODORSIL HUILE 550, RHODORSIL HUILE 510V500, RHODORSIL HUILE 710, sous les dénomination WACKER BELSIL PDM 20, PDM 200, PDM 1000 par la société WACKER.

La ou les silicones organomodifiées selon la présente invention sont de préférence présentes dans des concentrations allant de 0,1 à 40 % par rapport au poids total de la composition, et plus préférentiellement en une quantité allant de 0,5 à 20 %.

Parmi les composés du type sel de flavylium non substitués en position 3 conformes à l'invention, on utilisera de préférence ceux répondant à la formule (1) suivante :

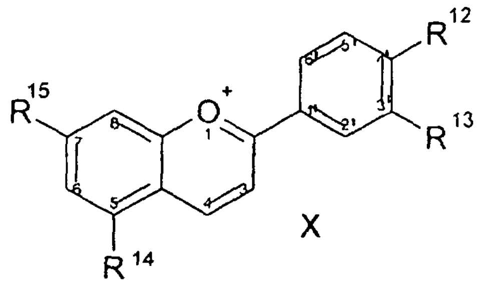

(1)

dans laquelle :

- $R^{12}$ désigne un radical OH ou alcoxy en $C_1$-$C_8$, linéaire ou ramifié, saturé ou insaturé,
- $R^{13}$, $R^{14}$, $R^{15}$, identiques ou différents, désignent H ou $R^{12}$, étant entendu qu'au moins un des radicaux $R^{12}$ à $R^{15}$ désigne OH,
- X- est un anion organique ou minéral et de préférence un dérivé d'acide minéral tel que par exemple un halogénure comme bromure ou chlorure ou bien un dérivé d'un acide organique comme par exemple acétate, borate, citrate, tartrate, lactate, bisulfate, sulfate, phosphate.

Les composés de formule (1) particulièrement préférés selon la présente invention sont choisis dans le groupe de ceux pour lesquels, dans la formule (1), $R^{12}$ désigne OH ou $OCH_3$.

On peut citer notamment parmi eux, les chlorures des composés suivants :

- 4', 5, 7-trihydroxyflavylium, communément dénommé "chlorure d'apigéninidine",
- 3', 4', 7-trihydroxyflavylium,
- 4'-hydroxyflavylium,
- 4', 7-dihydroxyflavylium,
- 3', 4'-dihydroxyflavylium,
- 3', 4'-dihydroxy-7-méthoxy-flavylium.
- 3',4', 5, 7-tétrahydroxyflavylium,
- 3', 4', 5', 5, 7-pentahydroxyflavylium.

Parmi ces composés, le chlorure d'apigéninidine (chlorure de 4', 5, 7-trihydroxyflavylium) et le chlorure de 3', 4', 7- trihydroxyflavylium sont encore plus particulièrement préférés.

Une forme particulière de l'invention consiste à utiliser le chlorure d'apigéninidine sous forme d'un extrait végétal, aisément préparé par extraction, et isolé, à partir de feuilles de Sorghum caudatum selon les procédés décrits dans les brevets CN 1064284A et CN1035512C ou toutes autres variantes de ces procédés .

Il peut aussi être extrait des tiges, des graines, ou des feuilles de Sorghum Bicolor, des pétales de Gesneria Fulgens, ainsi que des espèces Blechum Procerum et Sorgho en association avec du Colletotrichum Graminicola.

Une forme particulièrement préférée de l'invention est un extrait provenant des feuilles de Sorghum Bicolor obtenu par une extraction hydro-alcoolique en milieu acide à une température d'extraction allant de 30 à 40°C avec un rapport volume de solvant / masse de feuilles de Sorghum Bicolor allant de 10 à 30. Ledit extrait végétal de Sorghum titre environ 0,05 à 50% en poids de chlorure d'apigéninidine.

Les composés du type sel de flavylium, non substitués en position 3, et substitués par au moins un radical hydroxyle ou alcoxy, selon l'invention, peuvent être facilement obtenus par voie de synthèse, et à faible coût, notamment par la méthode bien connue de R. Robinson et D. D. Pratt J. Chem. Soc. 745 (1923). Ladite méthode implique de condenser une orthohydroxybenzaldéhyde ou ses dérivés de substitution sur une acétophénone ou ses dérivés de substitution, pour obtenir, en choisissant les substituants, les composés de formule (I) désirés.

En prenant comme exemple le chlorure d'apigéninidine (chlorure de 4', 5, 7-trihydroxyflavylium), le schéma de synthèse (i) peut être le suivant :

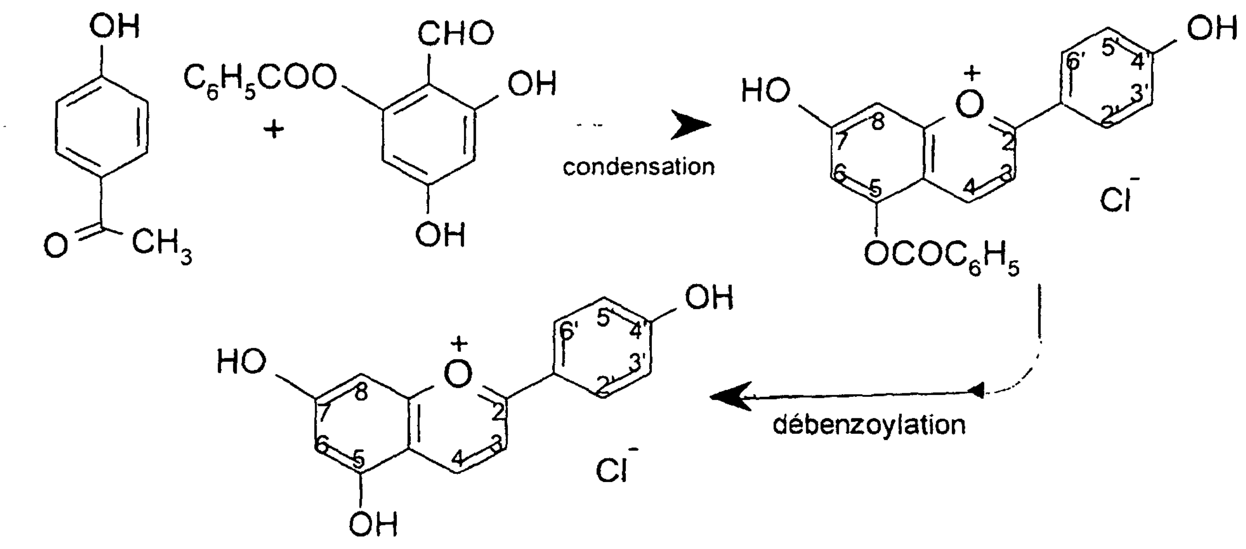


En prenant comme exemple le chlorure de 3', 4', 7- trihydroxyflavylium, le schéma de synthèse (ii) peut être le suivant :

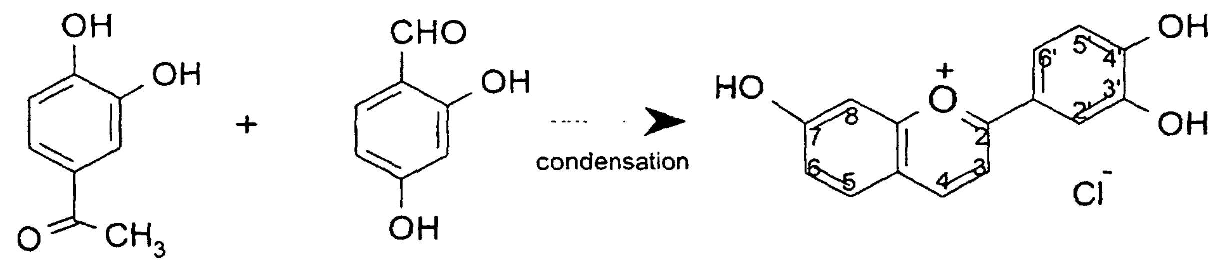


Diverses voies de synthèses, bien connues dans l'art antérieur, conduisent à l'apigéninidine.

Une méthode consiste, par exemple, à préparer, dans une première étape, la triméthylapigéninidine, par condensation du 4,6-diméthoxy-2-hydroxybenzaldéhyde commercial sur la 4-méthoxyacétophénone commerciale à 0°C en milieu éther anhydre, et saturation par HCl anhydre, pour obtenir après filtration un précipité rouge-orangé de triméthylapigéninidine. Dans une seconde étape, à hydrolyser la triméthylapigéninidine obtenue à l'étape précédente en chlorure d'apigéninidine, la réaction s'effectuant en milieu HI et phénol et AgCl en solution dans le méthanol. Une telle méthode de synthèse est décrite par R. Robinson et A. Robertson dans J. Chem. Soc. 1951 (1926) et 2196 (1927).

Une autre méthode consiste à condenser le 2, 4, 6-trihydroxybenzaldéhyde sur la 4-hydroxyacétophénone à 0°C en milieu solvant anhydre (acétate d'éthyle par exemple), et saturation par HCl anhydre, pour obtenir le chlorure d'apigéninidine. Une telle méthode est décrite par R. Robinson et A. Robertson dans J. Chem. Soc. 1528 (1928).

Une autre méthode de préparation du chlorure d'apigéninidine consiste à réduire une flavone, la naringénine, ou son dérivé triacétylé, par $NaBH_4$, puis à oxyder le produit obtenu par le chloranil (tétrachloro-1,4-benzoquinone). Ladite méthode est décrite par J. G. Sweeny et G. A. Iacobucci dans la revue Tetrahedron **33** 2923-2927 (1977).

La méthode la plus particulièrement préférée, selon la présente invention, consiste à condenser le 2,4-dihydroxy-6-benzoyloxybenzaldéhyde sur la 4-hydroxyacétophénone à 0°C en milieu acétate d'éthyle anhydre, à saturer par HCl anhydre, puis à débenzoyler le produit obtenu par la soude, afin d'obtenir le chlorure d'apigéninidine avec un rendement élevé, suivant le schéma (i) décrit ci-dessus. Ladite méthode est décrite par R. Robinson et J. C. Bell dans J. Chem. Soc. 813 (1934).

La concentration en composé du type sel de flavylium, tel que décrit selon la présente invention, est de préférence comprise entre environ 0,0001 et 10%, et encore plus préférentiellement entre 0,001 et 5% en poids, par rapport au poids total de la composition.

Les compositions conformes à la présente invention permettent d'obtenir au bout de 30 minutes après application sur une peau claire à raison de $2 mg/cm^2$ un assombrissement caractérisé dans le système de mesure colorimétrique (L*, a*, b*) par un $\Delta L^*$ allant de -0,5 à - 20. De façon préférentielle, $\Delta L^*$ variera de - 0,5 à - 15.

Les compositions conformes à la présente invention apportent au bout de 30 minutes après application sur la peau à raison de 2mg/cm$^2$ une coloration sur une peau claire définie dans le système de mesure colorimétrique (L*, a*, b*), par un rapport Δa*/Δb* allant de 0,5 à 3 et encore plus particulièrement allant de 0, 8 à 2.

Dans le système de mesure colorimétrique (L*, a*, b*) :

L* représente la luminance ou clarté, a* représente l'axe rouge-vert (-a*= vert, +a*= rouge) et b* représente l'axe jaune-bleu (-b*=bleu, +b*=jaune). Ainsi, a* et b* expriment la nuance de la peau.

ΔL* traduit l'assombrissement de la couleur : plus le ΔL* est négatif, plus la couleur s'est assombrie avec :

ΔL* = L* peau non colorée - L* peau colorée

Le rapport Δa*/Δb* traduit l'équilibre rouge/jaune et donc la nuance avec :

Δa* = a* peau non colorée - a* peau colorée

Δb* = b* peau non colorée - b* peau colorée

Selon la présente invention, on entend « par peau claire », des peaux non bronzées dont on peut définir les caractéristiques colorimétriques par leur angle ITA tel que défini dans la publication de A. Chardon et al. « Skin Colour Typology and Suntanning Pathways » et présentée au 16$^{th}$ IFSCC congress oct 8-10 1990 de New-York, et in *Int. J. Cosm. Sci.* **13** 191-208 (1991). Les peaux claires telles que définies dans cette classification ont un angle ITA compris entre 35 et 55.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

De façon particulièrement préférée, les compositions selon l'invention se présentent sous forme d'émulsion eau-dans-silicone et la silicone organomodifiée utilisée comporte de préférence des groupements oxyalkylénés (en particulier oxyéthylénés et/ou oxypropylénés) comme celles de formule (I) ou (II) définies précedemment.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent bien entendu contenir un ou plusieurs filtres organiques actifs dans l'UVA et/ou l'UVB. Ces filtres peuvent être notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone ; les dérivés de β,β'-diphénylacrylate, les dérivés de benzotriazole, les dérivés de benzimidazole ; les imadazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE19726184 et EP893119 ; les polymères filtres et silicones filtres tels que ceux décrits notamment dans la demande WO93/04665 ; les dimères dérivés d'α-alkylstyrène tels que ceux décrits dans la demande de brevet DE19855649 ; les dérivés de 4,4-diarylbutadiène tels que ceux décrits dans les demandes de brevet EP0967200 et DE19755649.

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions de l'invention peuvent comprendre en outre des adjuvants cosmétiques classiques notamment choisis parmi les corps gras, les solvants organiques, les épaississants, les adoucissants, les opacifiants, les stabilisants, les émollients, les agents anti-mousse, les agents hydratants, les parfums, les conservateurs, les polymères, les charges, les séquestrants, les propulseurs, les agents alcalinisants ou acidifiants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de paraffine, les huiles de silicone, volatiles ou non, les isoparaffines, les polyoléfines, les huiles fluorées et perfluorées. De même, les cires peuvent être choisies parmi les cires animales, fossiles, végétales, minérales ou de synthèse connues en soi.
Parmi les solvants organiques, on peut citer les alcools et polyols inférieurs.
Selon une forme particulièrement préférée, les compositions selon l'invention contiennent au moins 5% en poids par rapport au poids de la composition d'un ou plusieurs solvants polyhydroxylés. Ces derniers peuvent être choisis parmi les glycols et les éthers de glycol comme l'éthylène glycol, le propylène glycol, le butylène glycol, le dipropylène glycol ou le diéthylène glycol. Et plus particulièrement, les compositions selon l'invention contiennent un mélange d'au moins trois solvants polyhydroxylés différents et encore plus particulièrement un mélange constitué de propylène glycol, de butylène glycol, et de dipropylène glycol.
Bien entendu, l'homme de l'art veillera à choisir ce ou ces éventuels composés complémentaires et/ou leurs quantités de manière telle que les propriétés avantageuses, en particulier la rémanence à l'eau, la stabilité, attachées intrinsèquement aux émulsions conformes à l'invention ne soient pas, ou substantiellement pas, altérées par la ou les adjonctions envisagées.
Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).
Lorsque la composition cosmétique selon l'invention est utilisée pour la coloration de l'épiderme humain, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.
Comme indiqué en début de description, un autre objet de la présente invention réside dans l'utilisation d'une émulsion selon l'invention pour la fabrication de compositions cosmétiques pour la coloration de la peau et/ou des cheveux.
Un autre objet de la présente invention réside dans l'utilisation d'une silicone organomodifiée dans la fabrication d'une émulsion cosmétique ou dermatologique contenant au moins au moins un composé du type sel de flavylium non substitué en position 3, et substitué par au moins un radical hydroxyle ou alcoxy dans l'émulsion, en vue d'améliorer la stabilité et l'agrément cosmétique de la composition.
Des exemples concrets, mais nullement limitatifs, illustrant l'invention, vont maintenant être donnés.

**EXEMPLE 1 :**

On prépare un extrait de Sorghum Bicolor titrant à 20-30% de chlorure d'apigéninidine selon le procédé de préparation suivant :

un extrait provenant des feuilles de Sorghum Bicolor est obtenu par extraction hydro-alcoolique (éthanol 95°) en milieu acide (0.2% HCl) à une température d'extraction de 35°C avec un rapport volume de solvant / masse de feuilles de Sorghum Bicolor de 15. L' extrait végétal de Sorghum est séché à l'étuve 24 h à 40°C et tamisé à 200 µm.

Le rendement de cette extraction est de 22.42% en matière colorante.
Le titre de l'extrait ainsi obtenu est de 21% en poids de chlorure d'apigéninidine.
Cet exemple vise à montrer dans un premier temps la stabilité au stockage d'une composition A selon l'invention contenant l'extrait de Sorghum Bicolor dans un support émulsion (eau-dans-silicone) **contenant une silicone organomodifiée** par rapport à une composition B contenant l'extrait de Sorghum Bicolor dans un support émulsion (huile-dans-eau) **ne contenant pas de silicone organomodifiée**.
Cet exemple vise à montrer, dans un deuxième temps, que la composition A conforme à la présente invention appliquée sur une peau claire conduit rapidement à une coloration proche d'un bronzage naturel contrairement à une émulsion C de l'art antérieur **contenant une silicone organomodifiée mais contenant la DHA à la place de l'extrait de Sorghum Bicolor.**
La Demanderesse a réalisé les compositions suivantes :

Composition A (invention):

- Polydiméthyl/méthyl siloxane POE/POP(396/4) (OE/OP 18/18) A 10 % D5 10g
- Cyclopentadiméthylsiloxane 12.5g
- Mélange tocophérols naturels/huile de soja 0.1g
- Chlorure de sodium 2g
- Propylène glycol 23g
- Butylène glycol 5g
- Dipropylène glycol 10g
- Eau déminéralisee 35.959g
- Extrait de Sorghum Bicolor tel que préparé ci-dessus 0.7g
- Citrate trisodique 0.535g
- Acide citrique 0.206g

Composition B (hors invention)

- Mélange mono/distéarate de glycéryle/alcool cétylstéarylique oxyéthyléné 3g
- Alcool stéarylique 2.5g
- Mélange mono/distéarate de glycéryle/stéarate de polyéthylène glycol 2.5
- Mélange de para-hydrohybenzoates /phénoxy 2 éthanol 0.5g
- Polyisobutylène 1g
- Di-tertiobutyl 4-hydroxytoluène 0.5g
- Benzoate d'alcools $C_{12}/C_{15}$ 3g
- Extrait de Sorghum Bicolor tel que préparé ci-dessus 0.7g
- 1.3-butylène glycol 5g
- Propylène glycol 10g
- huile de vaseline 5g
- Acétate de DL-alpha-tocophéryle 0.25g
- Eau déminéralisée 66.05g

Les formules A et B ont été examinées d'abord macrocospiquement puis microscopiquement en lumière blanche et lumière polarisée après avoir subi un temps de conservation de 2 mois à température ambiante et dans une étuve à 45°C.

On observe qu'au bout de 2 mois de conservation la composition B est instable et présente des filaments, des précipités rouges et des bulles d'air tandis que la composition A a conservé son aspect initial.

**Comparaison avec une émulsion contenant la DHA :**

La Demanderesse a réalisé la composition suivante :

Composition C (hors invention):

- Polydiméthyl/méthyl siloxane POE/POP(396/4) (OE/OP 18/18) à 10 % D5 10g
- Cyclopentadiméthylsiloxane 12.5g
- Mélange tocophérols naturels/huile de soja 0.1g
- Dihydroxyacétone (DHA) 4g
- Chlorure de sodium 2g
- Propylène glycol 23g
- Butylène glycol 5g
- Dipropylène glycol 10g
- Citrate trisodique 0.535g
- Acide citrique 0.206g

- Eau déminéralisée 32.649g

Les compositions A et C ont été appliquées à raison de 2 $mg/cm^2$ sur une zone de 7 x 4,5 $cm^2$ délimitée sur le dos de 6. volontaires dont la couleur de peau caractérisée par l'angle ITA est compris entre 35 et 55.

Les cinq séries de mesures colorimétriques suivantes ont été effectuées à l'aide d'un colorimètre Minolta CR-300 :

- 1°) avant application de la composition,
- 2°) 30 minutes après l'application,
- 3°) 2 heures après application.
- 4°) 4 heures après application.
- 5°) 5 heures après application

Les résultats sont exprimés dans le système (L*, a*, b*) dans lequel L* représente la luminance, a* représente l'axe rouge-vert (-a* = vert, +a* = rouge) et b* représente l'axe jaune-bleu (-b* =bleu, +b* =jaune). Ainsi, a* et b* expriment la nuance de la peau.

Pour l'évaluation de l'intensité de la coloration, on s'intéresse à ΔL* qui traduit l'assombrissement de la couleur : plus ΔL* est négatif, plus la couleur s'est assombrie avec :

$$\Delta L^* = L^* \text{ peau non colorée} - L^* \text{ peau colorée}$$

Les résultats obtenus sont rassemblés dans le tableau (I) suivant :

Tableau (I) :

| | **Composition A (invention) ΔL*** | **Composition C (hors invention) ΔL*** |
|---|---|---|
| 30 minutes | - 9.8 | -0.4 |
| 2 heures | - 8.3 | -1.1 |
| 4 heures | - 8.1 | -2.5 |
| 5 heures | - 8.7 | -2.6 |

On constate ainsi que 30 minutes après l'application, la composition C, qui contient à titre d'agent de coloration de la peau, la DHA, n'a conféré qu'une très faible coloration à la peau, puisque la DHA n'a pas encore eu le temps d'agir (ΔL* = - 0,4). En revanche, la composition A selon l'invention a déjà conféré à la peau une coloration significative (ΔL* = - 8,2).

La composition C contenant la DHA ne permet pas d'obtenir au bout de 30 minutes un assombrissement comparable à celui de la composition A qui présente de plus une nuance de coloration constante pendant 5 heures.

**Revendications**

1. Emulsion cosmétique ou dermatologique, **caractérisée par le fait qu'**elle comporte :

    (b) au moins une phase aqueuse et
    (b) au moins une phase grasse ;
    (c) au moins un composé particulier du type sel de flavylium non substitué en position 3 et substitué par au moins un radical hydroxyle ou alcoxy, dans une quantité efficace permettant d'obtenir au bout de 30 minutes après application sur une peau claire à raison de 2$mg/cm^2$ un assombrissement de la couleur de peau caractérisé dans le système de mesure colorimétrique L* a* b* par un ΔL* allant de -0,5 à -20 ;
    (d) au moins une silicone organomodifiée.

2. Emulsion selon la revendication 1 où la silicone organomodifiée est choisie parmi :

    (1) les polyorganosiloxanes comportant des groupements oxyalkylénés

(2) les polyorganosiloxanes comportant des groupements alcoxylés
(3) les polyorganosiloxanes comportant des groupements anioniques du type 2-hydroxyalkylsulfonate ; 2-hydroxyalkylthiosulfate ;
(4) les polyorganosiloxanes comportant des groupements thiols ;
(5) les polyorganosiloxanes comportant des groupements anioniques du type carboxylique
(6) les polyorganosiloxanes comportant des groupements hydroxylés
(7) les polyorganosiloxanes comportant des groupements acyloxyalkyle
(8) les polyorganosiloxanes comportant des groupements aminés substitués ou non.
(9) les polyorganosiloxanes comportant des groupements hydroxyacylamino,
(10) les polyorganosiloxanes comportant des groupements de type aryle éventuellement substitué.

**3.** Emulsion selon la revendication 2, où les polyorganosiloxanes comportant des groupements oxyalkylénés répondent à l'une des formules suivantes :

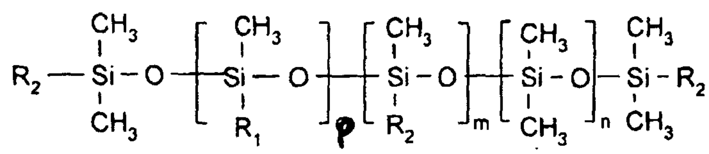 (I)

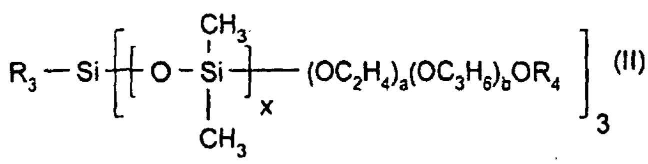 (II)

- $R_1$, identique ou différent, représente un radical alkyle, linéaire ou ramifié, en $C_1$-$C_{30}$ ou phényle.
- $R_2$, identique ou différent, $-C_cH_{2c}-(-O-C_2H_4)_a-(-O-C_3H_6)_b-(O-C_4H_8)_d-R_5$
- $R_3$, $R_4$, identiques ou différents, désignent un radical alkyle, linéaire ou ramifié, en $C_1$-$C_{12}$, et de préférence le radical méthyle,
- $R_5$, identique ou différent, est choisi parmi un atome d'hydrogène, un radical hydroxyle, un radical alkyle linéaire ou ramifié de 1 à 12 atomes de carbone, un radical alcoxy linéaire ou ramifié de 1 à 6 atomes de carbone, un radical acyloxy linéaire ou ramifié de 2 à 12 atomes de carbone, $NHCH_2CH_2COOM$, aminoalkyle éventuellement substitué sur l'amine, carboxyacyle en $C_1$-$C_{30}$, un groupement phosphono éventuellement substitué,
- $O-CO-(CH_2)_d-CO_2M$, $-NHCO(CH_2)_dOH$, $-NH_3Y$.
- M, identique ou différent, désigne un atome d'hydrogène, Na, K, Li, $NH_4$ ou une amine organique,
- a varie de 0 à 50,
- b varie de 0 à 50,
- c varie de 0 à 4,
- a + b est supérieur ou égal à 1,
- d varie de 0 à 10,
- m varie de 0 à 20,
- n varie de 0 à 500,
- p varie de 0 à 20,
- x varie de 1 à 100,
- Y représente un anion minéral ou organique monovalent.

**4.** Emulsion selon la revendication 2, où les polyorganosiloxanes comportant des groupements anioniques du type carboxylique répondent à la formule (III) suivante :

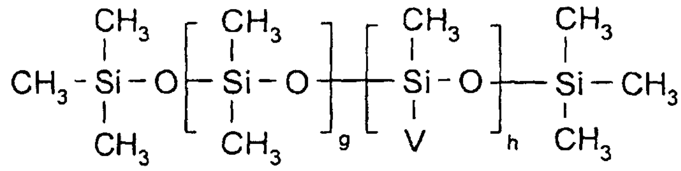 (III)

dans laquelle V est un radical $-(R^1O)_e-R^2-(OR^3)_f-COOM$, dans lequel $R^1$ et $R^3$ désignent indépendamment un radical alkylène linéaire ou ramifié ayant de 2 à 20 atomes de carbone, $R^2$ désigne un radical alkylène linéaire ou ramifié ayant de 1 à 50 atomes de carbone pouvant comprendre un groupement hydroxyle ; e représente 0 ou 1 et f est un nombre allant de 0 à 200 ; M désigne hydrogène, un métal alcalin ou alcalino-terreux, $NH_4$, un groupement ammonium quaternaire tel qu'un groupement mono-, di-, tri- ou tétra (alkyl $C_1$-$C_4$) ammonium ; g et h sont des nombres allant de 0 à 1000, la somme c+d allant de préférence de 2 à 1000.

**5.** Emulsion selon la revendication 2, où les polyorganosiloxanes comportant des groupements hydroxylés répondent à la formule (IV) suivante :

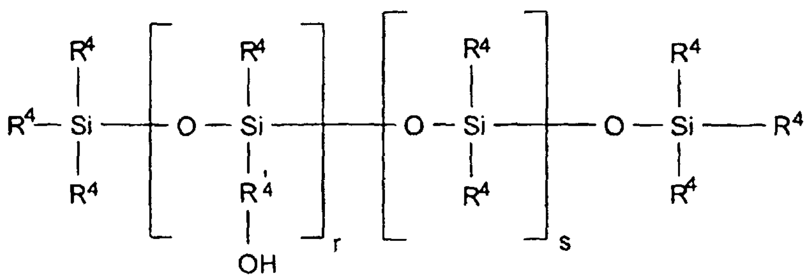 (IV)

dans laquelle les radicaux $R^4$ identiques ou différents sont choisis parmi les radicaux méthyle et phényle ; au moins 60 % en mole des radicaux $R^4$ désignant méthyle ; le radical $R'^4$ est un chaînon alkylène divalent hydrocarboné en $C_2$-$C_{18}$ ; r est compris entre 1 et 30 inclus ; s est compris entre 1 et 150 inclus .

**6.** Emulsion selon la revendication 2, où les polyorganosiloxanes comportant des groupements acyloxyalkyle répondant à la formule (V) :

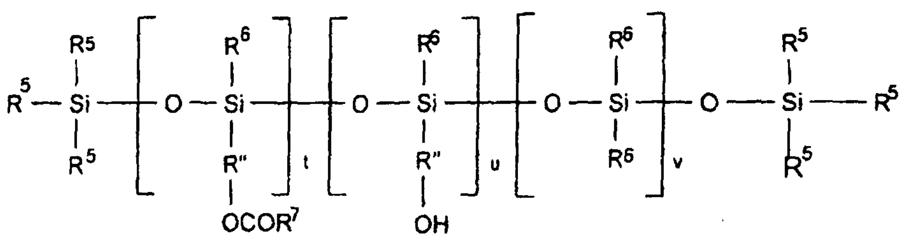 (V)

dans laquelle :

$R^5$ désigne un groupement méthyle, phényle, $-OCOR^7$, hydroxyle, un seul des radicaux $R^5$ par atome de silicium pouvant être OH ;
$R^6$ désigne méthyle, phényle ; au moins 60 % en proportion molaire de l'ensemble des radicaux $R^5$ et $R^6$ désignant méthyle ;
$R^7$ désigne alkyle ou alcényle en $C_8$-$C_{20}$ ;
R" désigne un radical alkylène hydrocarboné divalent, linéaire ou ramifié en $C_2$-$C_{18}$ ;
t est compris entre 1 et 120 inclus ;
u est compris entre 1 et 30 ;
v est égal à 0 ou est inférieur à 0,5 t, t + u étant compris entre 1 et 30 ; les polyorganosiloxanes de formule (V) peuvent contenir des groupements :

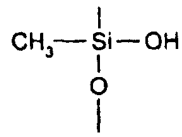

dans des proportions ne dépassant pas 15 % de la somme t + u + v.

**7.** Emulsion selon la revendication 2, où les polyorganosiloxanes comportant des groupements aminés substitués ou non sont choisis parmi :

(a) les polysiloxanes répondant à la formule :

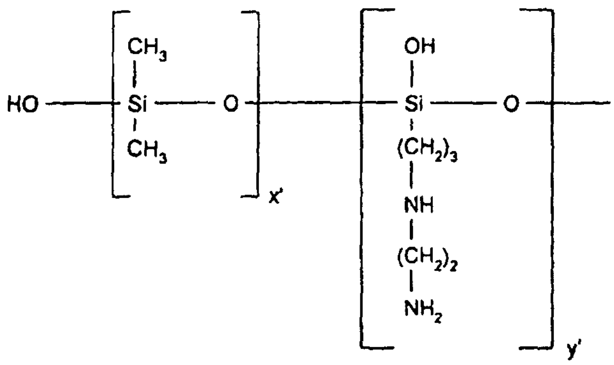

(VI)

dans laquelle x' et y' sont des nombres entiers tels que le poids moléculaire en nombre est compris entre 5 000 et 500 000 ;

(b) les polymères cationiques siliconés répondant à la formule :

$$R^8{}_iG_{3-i}\text{-}Si(OSiG_2)_k\text{-}(OSiG_jR^8{}_{2-j})_l\text{-}O\text{-}SiG_{3-i}\text{-}R^8{}_i \qquad \text{(VII)}$$

dans laquelle :

G est un atome d'hydrogène, ou un groupement phényle, OH, ou alkyle en $C_1$-$C_8$, par exemple méthyle,
i désigne le nombre 0 ou un nombre entier de 1 à 3, en particulier 0,
j désigne 0 ou 1, et en particulier 1,
k et l sont des nombres tels que la somme k+l peut varier notamment de 1 à 2 000 et en particulier de 50 à 150, k pouvant désigner un nombre de 0 à 1 999 et notamment de 49 à 149 et l pouvant désigner un nombre de 1 à 2 000, et notamment de 1 à 10 ;
$R^8$ est un radical monovalent de formule $-C_qH_{2q}L$ dans laquelle q est un nombre de 2 à 8 et L est un groupement aminé éventuellement quaternisé choisi parmi les groupements :
- $NR^9\text{-}CH_2\text{-}CH_2\text{-}N(R^9)_2$
- $N(R^9)_2$
- $N^{\oplus}(R^9)_3A^-$
- $N(R^9)\text{-}CH_2\text{-}CH_2\text{-}N^{\oplus}R^9H_2A^-$,

dans lesquels $R^9$ désigne hydrogène, phényle, benzyle, ou un radical hydrocarboné saturé monovalent, par exemple un radical alkyle ayant de 1 à 20 atomes de carbone et $A^-$ représente un ion halogénure ;

(c) les polymères cationiques siliconés répondant à la formule :

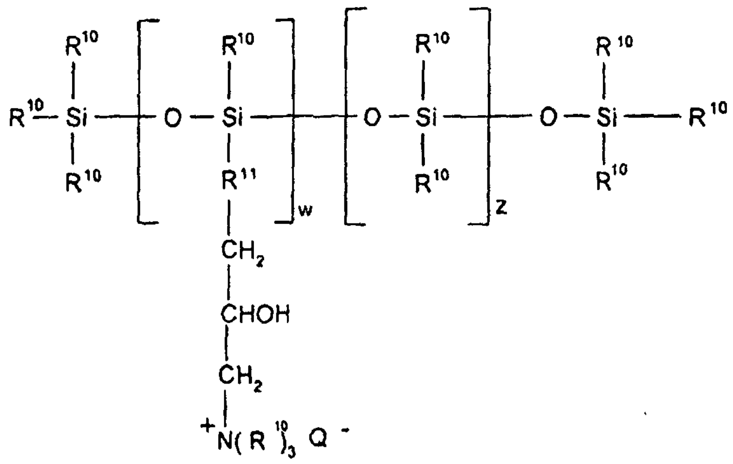

(VIII)

dans laquelle :

$R^{10}$ représente un radical hydrocarboné monovalent ayant de 1 à 18 atomes de carbone, et en particulier un radical alkyle en $C_1$-$C_{18}$, ou alcényle en $C_2$-$C_{18}$, par exemple méthyle ;
$R^{11}$ représente un radical hydrocarboné divalent, et en particulier un radical alkylène en $C_1$-$C_{18}$ ou un radical alkylèneoxy divalent en $C_1$-$C_{18}$, par exemple en $C_1$-$C_8$ ;
$Q^-$ est un ion halogénure ;
w représente une valeur statistique moyenne de 2 à 20 et en particulier de 2 à 8 ;
z représente une valeur statistique moyenne de 20 à 200 et en particulier de 20 à 50.

**8.** Emulsion selon l'une quelconque des revendications 1 à 7, où les silicones organomodifiées sont présentes dans des concentrations allant de 0,1 à 40 % par rapport au poids total de la composition, et plus préférentiellement en une quantité allant de 0,5 à 20 %.

**9.** Emulsion selon l'une quelconque des revendications 1 à 8, où le composé du type sel de flavylium non substitué en position 3 répond à la formule (1) suivante :

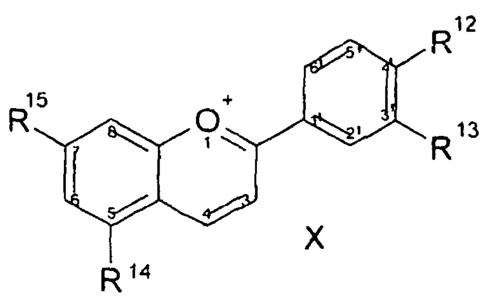

(1)

formule (I) dans laquelle :

- $R^{12}$ désigne un radical OH ou alcoxy en $C_1$-$C_8$, linéaire ou ramifié, saturé ou insaturé,
- $R^{13}$, $R^{14}$, $R^{15}$, identiques ou différents, désignent H ou $R^{12}$, étant entendu qu'au moins un des radicaux $R^{12}$ à $R^{15}$ désigne OH,
- $X^-$ est un anion organique ou minéral et de préférence du type halogénure ou dérivé d'un acide organique.

**10.** Emulsion selon la revendication 9, où dans la formule (I), $R^{12}$ désigne OH ou $OCH_3$.

**11.** Emulsion selon la revendication 9 ou 10, où le composé de formule (1) est choisi parmi les chlorures des composés suivants :

- 4', 5, 7-trihydroxyflavylium (ou chlorure d'apigéninidine) ,

- 3', 4', 7-trihydroxyflavylium,
- 4'-hydroxyflavylium,
- 4', 7-dihydroxyflavylium,
- 3', 4'-dihydroxyflavylium,
- 3', 4'-dihydroxy-7-méthoxy-flavylium,
- 3',4', 5, 7-tétrahydroxyflavinium,
- 3', 4', 5', 5, 7-pentahydroxyflavinum.

**12.** Emulsion selon la revendication 11, **caractérisée par le fait qu'**il s'agit du chlorure de 4', 5, 7-trihydroxyflavylium sous forme pure susceptible d'être obtenu par voie de synthèse.

**13.** Emulsion selon la revendication 11, **caractérisée par le fait qu'**il s'agit du chlorure de 4', 5, 7-trihydroxy flavylium, sous forme d'extrait végétal.

**14.** Emulsion selon la revendication 13, **caractérisée par le fait que** l'extrait végétal est un extrait végétal, obtenu à partir de feuilles de Sorghum caudatum, ; de tiges, de graines ou de feuilles de Sorghum Bicolor ; des pétales de Gesneria Fulgens, ainsi que des espèces Blechum Procerum et Sorgho en association avec du Colletotrichum Graminicola.

**15.** Emulsion selon la revendication 13, **caractérisée par le fait que** l'extrait végétal est un extrait de Sorghum Bicolor susceptible d'être obtenu par une extraction hydro-alcoolique acide à une température d'extraction allant de 30 à 40°C avec un rapport volume de solvant / masse de feuilles de Sorghum Bicolor allant de 10 à 30.

**16.** Emulsion selon la revendication 15, **caractérisée par le fait que** l'extrait de Sorghum Bicolor titre de 0,05 à 50% en poids de chlorure de 4', 5, 7-trihydroxy flavylium.

**17.** Emulsion selon l'une quelconque des revendications 1 à 16, où la concentration en composé du type sel de flavylium varie de 0,0001 à 10%, par rapport au poids total de la composition.

**18.** Emulsion selon la revendication 17, où la concentration en composé du type sel de flavylium, varie de 0,001 à 5% en poids, par rapport au poids total de la composition.

**19.** Emulsion selon l'une quelconque des revendications 1 à 18, **caractérisée par le fait qu'**elle se présente sous forme d'émulsion eau-dans-silicone.

**20.** Emulsion selon la revendication 19, où la silicone organomodifiée est choisi parmi celles comportant des groupements oxyalkylénés telles que définies dans la revendication 2 ou 3.

**21.** Emulsion selon l'une quelconque des revendications 1 à 20, contenant au moins 5% en poids par rapport au poids de la composition d'un ou plusieurs solvants polyhydroxylés.

**22.** Emulsion selon la revendication 21, où les solvants polyhydroxylés sont choisis parmi les glycols et les éthers de glycol.

**23.** Emulsion selon la revendication 21 ou 22, où les solvants polyhydroxylés sont choisis parmi l'éthylène glycol, le propylène glycol, le butylène glycol, le dipropylène glycol ou le diéthylène glycol et leurs mélanges.

**24.** Emulsion selon l'une quelconque des revendications 21 à 23, contenant un mélange d'au moins trois solvants polyhydroxylés différents .

**25.** Emulsion selon la revendication 24, contenant un mélange constitué de propylène glycol, de butylène glycol et de dipropylène glycol.

**26.** Procédé de traitement cosmétique de la peau destiné à lui conférer une coloration artificielle proche du bronzage naturel, **caractérisé en ce qu'**il consiste à appliquer sur celle-ci une quantité efficace d'une émulsion telle que définie à l'une quelconque des revendications 1 à 26.

**27.** Utilisation d'au moins un composé du type sel de flavylium, non substitué en position 3, et substitué par au moins

un radical hydroxyle ou alcoxy, obtenu par voie de synthèse, ou à partir d'un extrait végétal, ou d'un extrait végétal enrichi le contenant, et d'au moins une silicone organomodifiée tels que définis dans les revendications précédentes dans des compositions cosmétiques et/ou dermatologiques dans le but de conférer à la peau une coloration artificielle proche du bronzage naturel.

## Claims

**1.** Cosmetic or dermatological emulsion, **characterized in that** it comprises:

(a) at least one aqueous phase and
(b) at least one fatty phase;
(c) at least one particular flavylium salt compound which is unsubstituted in position 3, and which is substituted with at least one hydroxyl or alkoxy radical, in an amount which is effective for obtaining, 30 minutes after application to a fair skin at a rate of 2 mg/cm$^2$, a darkening of the skin colour **characterized in** the L*a*b* colorimetric measuring system by a ΔL* ranging from -0.5 to -20;
(d) at least one organomodified silicone.

**2.** Emulsion according to Claim 1, in which the organomodified silicone is chosen from:

(1) polyorganosiloxanes comprising oxyalkylenated groups;
(2) polyorganosiloxanes comprising alkoxylated groups;
(3) polyorganosiloxanes comprising anionic groups such as 2-hydroxyalkylsulphonate; 2-hydroxyalkylthiosulphate;
(4) polyorganosiloxanes comprising thiol groups;
(5) polyorganosiloxanes comprising anionic groups of carboxylic type;
(6) polyorganosiloxanes comprising hydroxylated groups;
(7) polyorganosiloxanes comprising acyloxyalkyl groups;
(8) polyorganosiloxanes comprising substituted or unsubstituted amine groups;
(9) polyorganosiloxanes comprising hydroxyacylamino groups;
(10) polyorganosiloxanes comprising optionally substituted aryl groups.

**3.** Emulsion according to Claim 2, in which the polyorganosiloxanes comprising oxyalkylenated groups correspond to one of the following formulae:

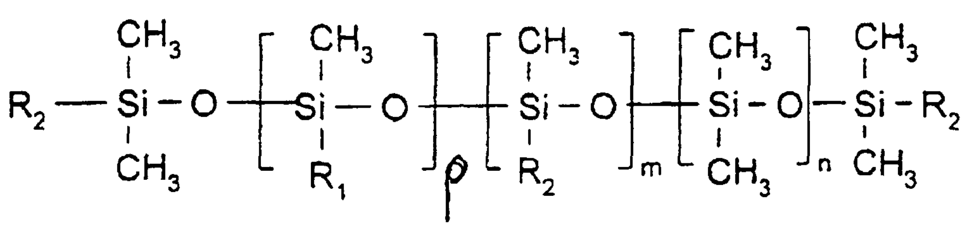

(I)

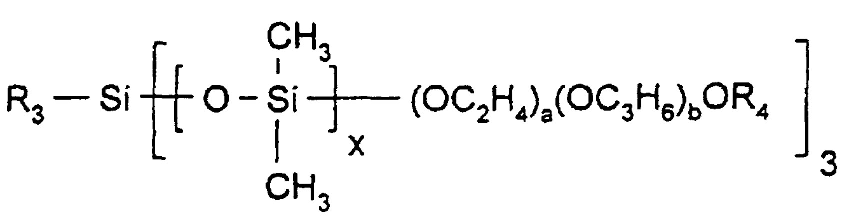

(II)

- $R_1$, which may be identical or different, represents a linear or branched $C_1$-$C_{30}$ alkyl radical or phenyl radical,
- $R_2$, which may be identical or different, represents $-C_cH_{2c}-(-O-C_2H_4)_a-(-O-C_3H_6)_b-(O-C_4H_8)_d-R_5$
- $R_3$ and $R_4$, which may be identical or different, denote a linear or branched $C_1$-$C_{12}$ alkyl radical and preferably a methyl radical,

- $R_5$, which may be identical or different, is chosen from a hydrogen atom, a hydroxyl radical, a linear or branched alkyl radical of 1 to 12 carbon atoms, a linear or branched alkoxy radical of 1 to 6 carbon atoms, a linear or branched acyloxy radical of 2 to 12 carbon atoms, $NHCH_2CH_2COOM$, aminoalkyl optionally substituted on the amine, carboxy($C_1$-$C_{30}$) acyl, an optionally substituted phosphono group, $-O-CO-(CH_2)_d-CO_2M$, $-NHCO(CH_2)_dOH$ or $-NH_3Y$,
- M, which may be identical or different, denotes a hydrogen atom, Na, K, Li, $NH_4$ or an organic amine,
- a ranges from 0 to 50,
- b ranges from 0 to 50,
- c ranges from 0 to 4,
- a + b is greater than or equal to 1,
- d ranges from 0 to 10,
- m ranges from 0 to 20,
- n ranges from 0 to 500,
- p ranges from 0 to 20,
- x ranges from 1 to 100,
- Y represents a mineral or organic monovalent anion.

**4.** Emulsion according to Claim 2, in which the polyorganosiloxanes comprising anionic groups of the carboxylic type correspond to formula (III) below:

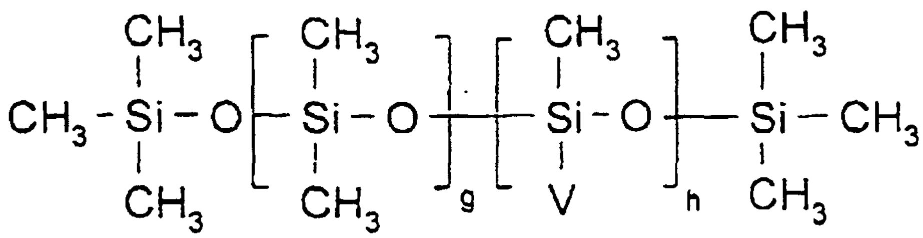

(III)

in which V is a radical $-(R^1O)_e-R^2-(OR^3)_f-COOM$, in which $R^1$ and $R^3$ independently denote a linear or branched alkylene radical containing from 2 to 20 carbon atoms, $R^2$ denotes a linear or branched alkylene radical containing from 1 to 50 carbon atoms which may comprise a hydroxyl group; e represents 0 or 1 and f is a number ranging from 0 to 200; M denotes hydrogen, an alkali metal or alkaline-earth metal, $NH_4$ or a quaternary ammonium group such as a mono-, di-, tri- or tetra($C_1$-$C_4$ alkyl)ammonium group; g and h are numbers ranging from 0 to 1000, the sum c+d preferably ranging from 2 to 1000.

**5.** Emulsion according to Claim 2, in which the polyorganosiloxanes comprising hydroxylated groups correspond to formula (IV) below:

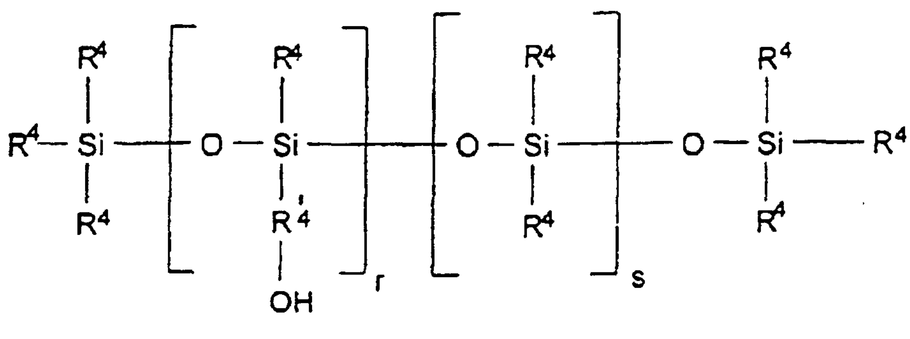

(IV)

in which the radicals $R^4$, which may be identical or different, are chosen from methyl and phenyl radicals, at least 60 mol% of the radicals $R^4$ denoting methyl; the radical $R'^4$ is a divalent hydrocarbon-based $C_2$-$C_{18}$ alkylene chain unit; r is between 1 and 30 inclusive; s is between 1 and 150 inclusive.

**6.** Emulsion according to Claim 2, in which the polyorganosiloxanes comprising acyloxyalkyl groups correspond to formula (V):

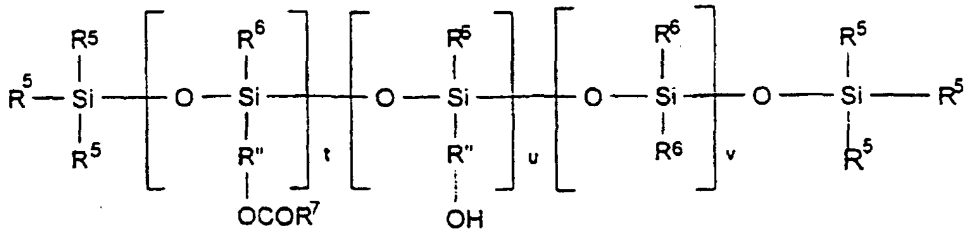 (V)

in which:

$R^5$ denotes a methyl, phenyl, $-OCOR^7$ or hydroxyl group, only one of the radicals $R^5$ per silicon atom possibly being OH;
$R^6$ denotes methyl or phenyl, at least 60 mol% of all of the radicals $R^5$ and $R^6$ denoting methyl;
$R^7$ denotes $C_8$-$C_{20}$ alkyl or alkenyl;
R" denotes a linear or branched divalent hydrocarbon-based $C_2$-$C_{18}$ alkylene radical;
t is between 1 and 120 inclusive;
u is between 1 and 30;
v is equal to 0 or is less than 0.5 t, t + u being between 1 and 30; the polyorganosiloxanes of formula (V) may contain groups:

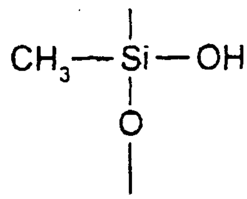

in proportions not exceeding 15% of the sum t+u+v.

7. Emulsion according to Claim 2, in which the polyorganosiloxanes comprising substituted or unsubstituted amine groups are chosen from:

(a) the polysiloxanes corresponding to the formula:

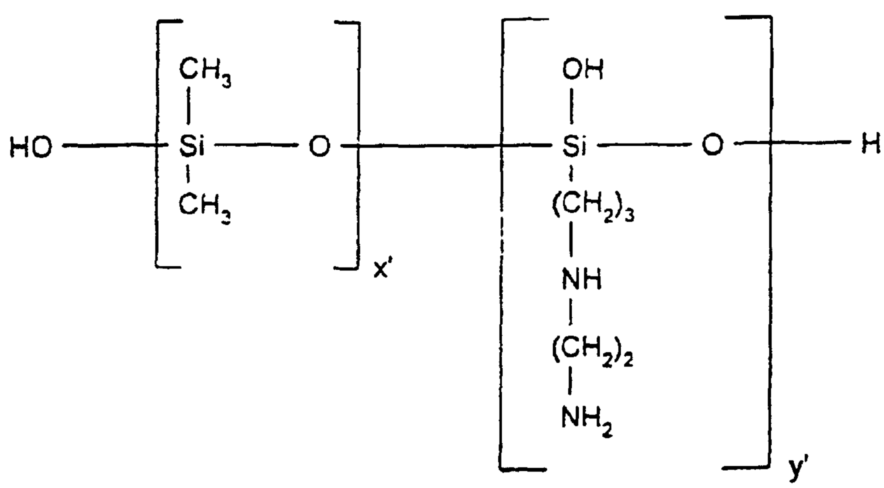 (VI)

in which x' and y' are integers such that the number-average molecular weight is between 5000 and 500,000;
(b) the cationic silicone polymers corresponding to the formula:

$$R^8{}_iG_{3-i}\text{-}Si(OSiG_2)_k\text{-}(OSiG_jR^8{}_{2-j})_l\text{-}O\text{-}SiG_{3-i}\text{-}R^8{}_i \quad (VII)$$

in which:

G is a hydrogen atom or a phenyl, OH or $C_1$-$C_8$ alkyl group, for example methyl,
i denotes the number 0 or an integer from 1 to 3, in particular 0,
j denotes 0 or 1 and in particular 1,
k and 1 are numbers such that the sum (k+l) may range especially from 1 to 2000 and in particular from 50 to 150, k possibly denoting a number from 0 to 1999 and especially from 49 to 149 and l possibly denoting a number from 1 to 2000 and especially from 1 to 10;
$R^8$ is a monovalent radical of formula $-C_qH_{2q}L$ in which q is a number from 2 to 8 and L is an optionally quaternized amine group chosen from groups:
$-NR^9\text{-}CH_2\text{-}CH_2\text{-}N(R^9)_2$
$-N(R^9)_2$
$-N^{\oplus}(R^9)_3A^-$
$-N(R^9)\text{-}CH_2\text{-}CH_2\text{-}N^{\oplus}R^9H_2A^-$,

in which $R^9$ denotes hydrogen, phenyl, benzyl or a saturated monovalent hydrocarbon-based radical, for example an alkyl radical containing from 1 to 20 carbon atoms and $A^-$ represents a halide ion;
(c) the cationic silicone polymers corresponding to the formula:

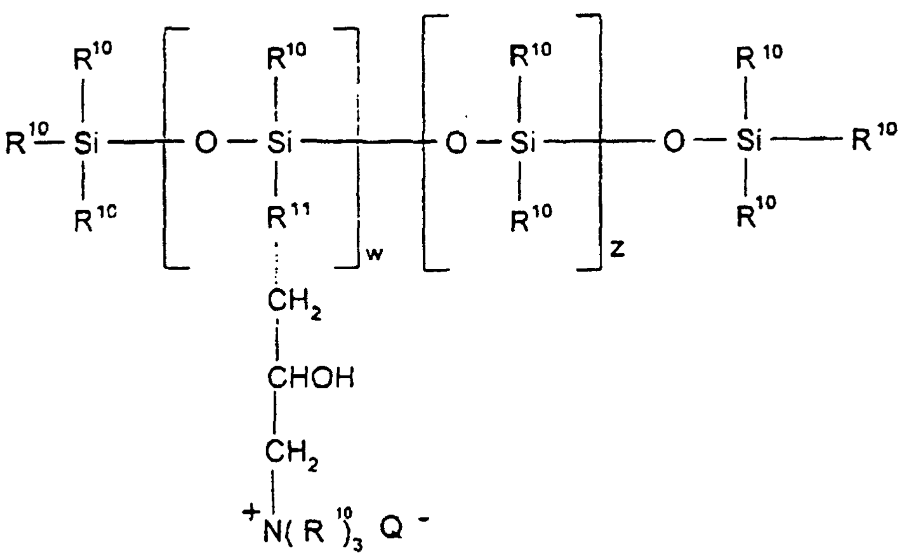
(VIII)

in which:

$R^{10}$ represents a monovalent hydrocarbon-based radical containing from 1 to 18 carbon atoms, and in particular a $C_1$-$C_{18}$ alkyl or $C_2$-$C_{18}$ alkenyl radical, for example methyl;
$R^{11}$ represents a divalent hydrocarbon-based radical, and in particular a $C_1$-$C_{18}$ alkylene radical or a divalent $C_1$-$C_{18}$, for example $C_1$-$C_8$, alkylenoxy radical;
$Q^-$ is a halide ion;
w represents an average statistical value from 2 to 20 and in particular from 2 to 8;
z represents an average statistical value from 20 to 200 and in particular from 20 to 50.

**8.** Emulsion according to any one of Claims 1 to 7, in which the organomodified silicones are present in concentrations ranging from 0.1% to 40% relative to the total weight of the composition and more preferably in an amount ranging from 0.5% to 20%.

**9.** Emulsion according to any one of Claims 1 to 8, in which the flavylium salt compound which is unsubstituted in position 3 corresponds to formula (1) below:

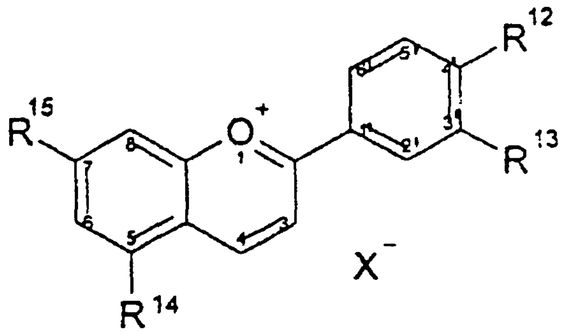

(1)

in which formula (1):

- $R^{12}$ denotes an OH or linear or branched, saturated or unsaturated $C_1$-$C_8$ alkoxy radical,
- $R^{13}$, $R^{14}$ and $R^{15}$, which may be identical or different, denote H or $R^{12}$, it being understood that at least one of the radicals $R^{12}$ to $R^{15}$ denotes OH,
- $X^-$ is an organic or mineral anion, preferably such as a halide or an organic acid derivative.

**10.** Emulsion according to Claim 9, in which, in formula (1), $R^{12}$ denotes OH or $OCH_3$.

**11.** Emulsion according to Claim 9 or 10, in which the compound of formula (1) is chosen from chlorides of the following compounds:

- 4',5,7-trihydroxyflavylium (or apigeninidine chloride),
- 3',4',7-trihydroxyflavylium,
- 4'-hydroxyflavylium,
- 4',7-dihydroxyflavylium,
- 3',4'-dihydroxyflavylium,
- 3',4'-dihydroxy-7-methoxyflavylium,
- 3',4',5,7-tetrahydroxyflavylium,
- 3',4',5',5,7-pentahydroxyflavylium.

**12.** Emulsion according to Claim 11, **characterized in that** it is 4',5,7-trihydroxyflavylium chloride in pure form which may be obtained synthetically.

**13.** Emulsion according to Claim 11, **characterized in that** it is 4',5,7-trihydroxyflavylium chloride, in the form of a plant extract.

**14.** Emulsion according to Claim 13, **characterized in that** the plant extract is a plant extract obtained from leaves of Sorghum caudatum; from stems, seeds or leaves of Sorghum bicolor; from petals of Gesneria fulgens, and also from the species Blechum procerum and Sorghum in combination with Colletotrichum graminicola.

**15.** Emulsion according to Claim 13, **characterized in that** the plant extract is an extract of Sorghum bicolor which may be obtained by an acidic aqueous-alcoholic extraction at an extraction temperature ranging from 30°C to 40°C with a ratio of the volume of solvent to the mass of Sorghum bicolor leaves ranging from 10 to 30.

**16.** Emulsion according to Claim 15, **characterized in that** the extract of Sorghum bicolor has a titre of from 0.05% to 50% by weight of 4',5,7-trihydroxyflavylium chloride.

**17.** Emulsion according to any one of Claims 1 to 16, in which the concentration of flavylium salt compound ranges from 0.0001% to 10% relative to the total weight of the composition.

**18.** Emulsion according to Claim 17, in which the concentration of flavylium salt compound ranges from 0.001% to 5% by weight relative to the total weight of the composition.

**19.** Emulsion according to any one of Claims 1 to 18, **characterized in that** it is in the form of a water-in-silicone

emulsion.

**20.** Emulsion according to Claim 19, in which the organomodified silicone is chosen from those comprising oxyalkylenated groups as defined in Claim 2 or 3.

**21.** Emulsion according to any one of Claims 1 to 20, containing at least 5% by weight, relative to the weight of the composition, of one or more polyhydroxylated solvents.

**22.** Emulsion according to Claim 21, in which the polyhydroxylated solvents are chosen from glycols and glycol ethers.

**23.** Emulsion according to Claim 21 or 22, in which the polyhydroxylated solvents are chosen from ethylene glycol, propylene glycol, butylene glycol, dipropylene glycol and diethylene glycol, and mixtures thereof.

**24.** Emulsion according to any one of Claims 21 to 23, containing a mixture of at least three different polyhydroxylated solvents.

**25.** Emulsion according to Claim 24, containing a mixture consisting of propylene glycol, butylene glycol and dipropylene glycol.

**26.** Cosmetic treatment process for the skin which is intended to give it an artificial coloration close to that of a natural tan, **characterized in that** it consists in applying to the skin an effective amount of an emulsion as defined in any one of Claims 1 to 25.

**27.** Use of at least one flavylium salt compound which is unsubstituted in position 3, and which is substituted with at least one hydroxyl or alkoxy radical, obtained synthetically or from a plant extract or from an enriched plant extract containing it, and of at least one organomodified silicone as defined in the preceding claims, in cosmetic and/or dermatological compositions with the aim of giving the skin an artificial coloration close to that of a natural tan.

## Patentansprüche

**1.** Kosmetische oder dermatologische Emulsion, **dadurch gekennzeichnet, dass** sie enthält:

(a) mindestens eine wässrige Phase und
(b) mindestens eine Fettphase;
(c) mindestens eine spezielle Verbindung vom Typ der Flavyliumsalze, die in 3-Stellung nicht substituiert und mit mindestens einer Hydroxygruppe oder Alkoxygruppe substituiert sind, in einer solchen Menge, dass 30 Minuten nach dem Auftragen in einer Menge von 2 $mg/cm^2$ auf helle Haut ein Dunklerwerden der Hautfarbe erreicht wird, das im colorimetrischen L* a* b*-System durch ein $\Delta L^*$ von -0,5 bis -20 gekennzeichnet ist, und
(d) mindestens ein organomodifiziertes Silicon.

**2.** Emulsion nach Anspruch 1, wobei das organomodifizierte Silicon ausgewählt ist unter:

(1) Polyorganosiloxanen mit Alkylenoxidgruppen;
(2) Polyorganosiloxanen mit Alkoxygruppen;
(3) Polyorganosiloxanen mit anionischen Gruppen vom Typ 2-Hydroxyalkylsulfonat; 2-Hydroxyalkylthiosulfat;
(4) Polyorganosiloxanen mit Thiolgruppen;
(5) Polyorganosiloxanen mit anionischen Gruppen vom Carboxytyp;
(6) Polyorganosiloxanen mit Hydroxygruppen;
(7) Polyorganosiloxanen mit Acyloxyalkylgruppen;
(8) Polyorganosiloxanen mit substituierten oder unsubstituierten Aminogruppen;
(9) Polyorganosiloxanen mit Hydroxyacylaminogruppen;
(10) Polyorganosiloxanen mit Gruppen vom gegebenenfalls substituierten Aryltyp.

**3.** Emulsion nach Anspruch 2, wobei die Polyorganosiloxane mit Alkylenoxidgruppen einer der folgenden Formeln entsprechen:

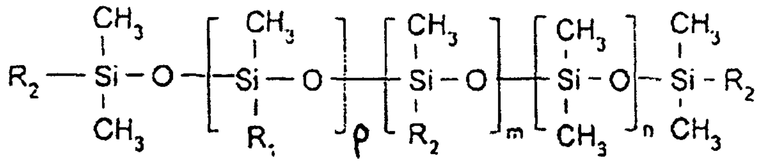 (I)

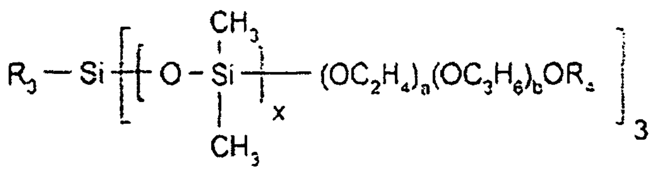 (II)

worin bedeuten:

- die Gruppen $R_1$, die gleich oder verschieden sind, eine geradkettige oder verzweigte $C_{1-30}$-Alkylgruppe oder Phenyl,
- die Gruppen $R_2$, die gleich oder verschieden sind,
- $C_cH_{2c}$-(-O-$C_2H_4$)$_a$-(-O-$C_2H_4$)$_a$-(-O-$C_3H_6$)$_b$-(O-$C_4H_8$)$_d$-$R_5$
- die Gruppen $R_3$ und $R_4$, die gleich oder verschieden sind, eine geradkettige oder verzweigte $C_{1-12}$-Alkylgruppe und vorzugsweise die Methylgruppe,
- die Gruppen Rs, die gleich oder verschieden sind, ein Wasserstoffatom, eine Hydroxygruppe, eine geradkettige oder verzweigte Alkylgruppe mit 1 bis 12 Kohlenstoffatomen, eine geradkettige oder verzweigte Alkoxygruppe mit 1 bis 6 Kohlenstoffatomen, eine geradkettige oder verzweigte Acyloxygruppe mit 2 bis 12 Kohlenstoffatomen, $NHCH_2CH_2COOM$, eine Aminoalkylgruppe, deren Aminogruppe gegebenenfalls substituiert ist, eine $C_{1-30}$-Carboxyacylgruppe, eine gegebenenfalls substituierte Phosphonogruppe, -O-CO-$(CH_2)_d$-$CO_2M$, -NHCO$(CH_2)_d$OH oder -$NH_3Y$,
- die Gruppen M, die gleich oder verschieden sind, ein Wasserstoffatom, Na, K, Li, $NH_4$ oder ein organisches Amin,
- a liegt im Bereich von 0 bis 50,
- b liegt im Bereich von 0 bis 50,
- c liegt im Bereich von 0 bis 4,
- a + b bedeutet 1 oder liegt über 1,
- d liegt im Bereich von 0 bis 10,
- m liegt im Bereich von 0 bis 20,
- n liegt im Bereich von 0 bis 500,
- p liegt im Bereich von 0 bis 20,
- x liegt im Bereich von 1 bis 100,
- Y bedeutet ein einwertiges anorganisches oder organisches Anion.

**4.** Emulsion nach Anspruch 2, wobei die Polyorganosiloxane mit anionischen Gruppen vom Carboxytyp der folgenden Formel (III) entsprechen:

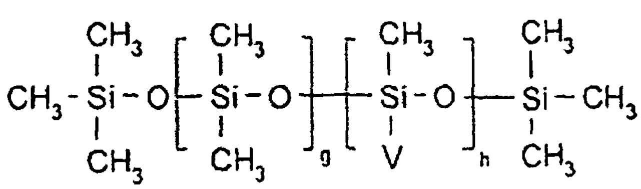 (III)

worin V eine Gruppe -$(R^1O)_e$-$R^2$-$(OR^3)_f$-COOM bedeutet, wobei $R^1$ und $R^3$ unabhängig voneinander eine gerad-

kettige oder verzweigte Alkylengruppe mit 2 bis 20 Kohlenstoffatomen bedeuten, $R^2$ eine geradkettige oder verzweigte Alkylengruppe mit 1 bis 50 Kohlenstoffatomen ist, die eine Hydroxygruppe enthalten kann; e 0 oder 1 bedeutet und f eine Zahl im Bereich von 0 bis 200 ist; M Wasserstoff, ein Alkalimetall, ein Erdalkalimetall, $NH_4$ oder eine quartäre Ammoniumgruppe bedeutet, beispielsweise eine Mono-, Di-, Tri- oder Tetra($C_{1-4}$-alkyl)ammoniumgruppe; und g und h Zahlen von 0 bis 1000 bedeuten, wobei die Summe c+d vorzugsweise im Bereich von 2 bis 1000 liegt.

**5.** Emulsion nach Anspruch 2, wobei die Polyorganosiloxane mit Hydroxygruppen der folgenden Formel (IV) entsprechen:

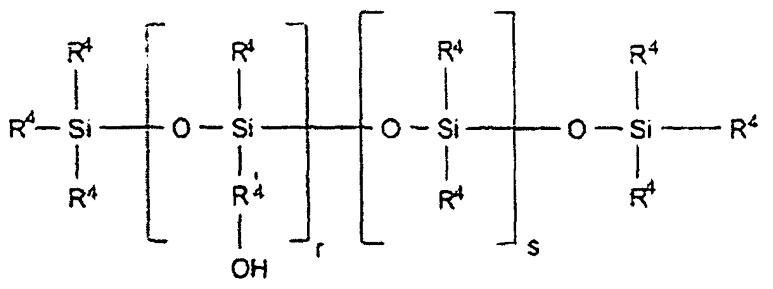

(IV)

worin die Gruppen $R^4$, die gleich oder verschieden sind, unter den Gruppen Methyl und Phenyl ausgewählt sind, wobei mindestens 60 Mol-% der Gruppen $R^4$ Methyl bedeuten; die Gruppe $R'^4$ ist eine zweiwertige Alkylenkette mit 2 bis 18 Kohlenstoffatomen; r liegt im Bereich von 1 bis 30, und s liegt im Bereich von 1 bis 150.

**6.** Emulsion nach Anspruch 2, wobei die Polyorganosiloxane mit Acyloxyalkylgruppen der folgenden Formel (V) entsprechen:

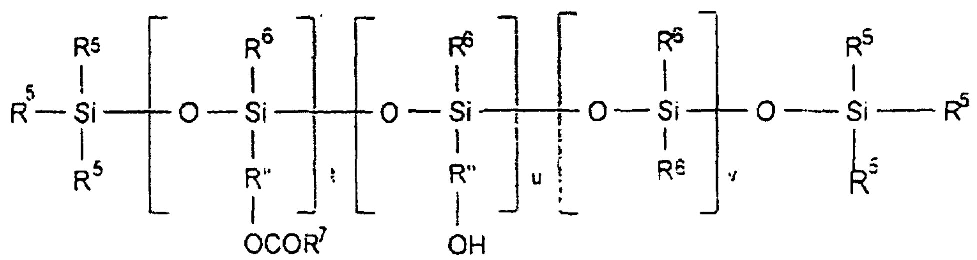

(V)

worin bedeuten:

$R^5$ Methyl, Phenyl, $-OCOR^7$, Hydroxy, wobei nur eine Gruppen $R^5$ pro Siliciumatom OH bedeuten kann;
$R^6$ Methyl, Phenyl; wobei mindestens 60 Mol-% der gesamten Gruppen $R^5$ und $R^6$ Methyl bedeuten;
$R^7$ Alkyl oder Alkenyl mit 8 bis 20 Kohlenstoffatomen;
R" eine geradkettige oder verzweigte, zweiwertige Alkylengruppe mit 2 bis 18 Kohlenstoffatomen;
t liegt im Bereich von 1 bis 120;
u liegt im Bereich von 1 bis 30;
v bedeutet 0 oder liegt unter 0,5 t, wobei t + u im Bereich von 1 bis 30 liegt; die Polyorganosiloxane der Formel (V) können die folgenden Gruppen:

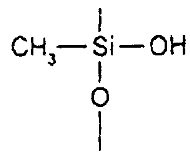

in Anteilen enthalten, die 15 % der Summe t + u + v nicht übersteigen.

**7.** Emulsion nach Anspruch 2, wobei die Polyorganosiloxane mit substituierten oder unsubstituierten Aminogruppen ausgewählt sind unter:

(a) Polysiloxanen der folgenden Formel:

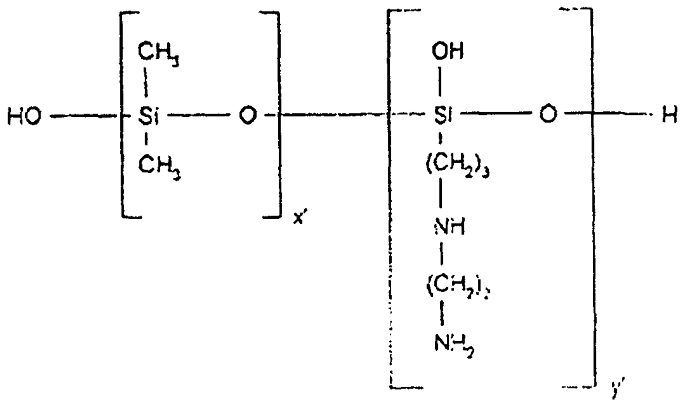

(VI)

worin x' und y' ganze Zahlen bedeuten, die von der Molmasse abhängen und im Allgemeinen so gewählt sind, dass die zahlenmittlere Molmasse im Bereich von 5.000 bis 500.000 liegt;

(b) kationischen siliconhaltigen Polymeren der folgenden Formel:

$$R^8{}_iG_{3-i}\text{-Si}(OSiG_2)_k\text{-}(OSiGR^8{}_{2-j)})_l\text{-O-SiG}_{3-i}\text{-}R^8{}_i \quad \text{(VII)}$$

worin bedeuten:

G ein Wasserstoffatom, eine Phenylgruppe, OH oder $C_{1-8}$-Alkyl, beispielsweise Methyl,
i 0 oder eine ganze Zahl von 1 bis 3 und insbesondere 0,
j 0 oder 1 und insbesondere 1,
k und l Zahlen, die so gewählt sind, dass die Summe (k + l) insbesondere im Bereich von 1 bis 2.000 und besonders 50 bis 150 liegen kann, wobei k eine Zahl von 0 bis 1.999 und insbesondere 49 bis 149 bedeuten und l eine Zahl von 1 bis 2.000 und insbesondere 1 bis 10 bedeuten kann,
$R^8$ eine einwertige Gruppe der Formel $-C_qH_{2q}L$, wobei q eine Zahl von 2 bis 8 ist und L eine gegebenenfalls quarternisierte aminierte Gruppe ist, die unter den folgenden Gruppen ausgewählt ist:
$-NR^9-CH_2-CH_2-N(R^9)_2$
$-N(R^9)_2$
$-N^{\oplus}(R^9)_3A^-$
$-N(R^9)-CH_2-CH_2-N^{\oplus}R^9H_2A^-$,

wobei $R^9$ Wasserstoff, Phenyl, Benzyl oder eine gesättigte einwertige Kohlenwasserstoffgruppe bedeutet, beispielsweise eine Alkylgruppe mit 1 bis 20 Kohlenstoffatomen, und $A^-$ ein Halogenidion ist,

(c) kationischen siliconierten Polymeren der folgenden Formel:

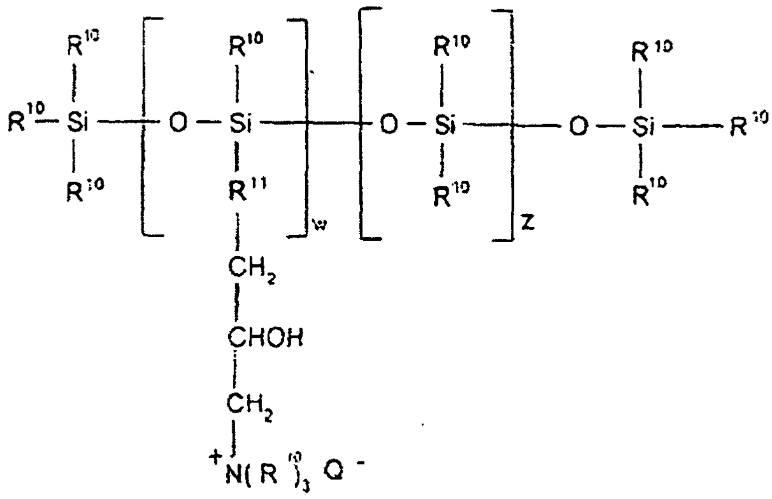

(VIII)

worin bedeuten:

$R^{10}$ bedeutet eine einwertige Kohlenwasserstoffgruppe mit 1 bis 18 Kohlenstoffatomen, insbesondere $C_{1-18}$-Alkyl oder $C_{2-18}$-Alkenyl, beispielsweise Methyl;
$R^{11}$ bedeutet eine zweiwertige Kohlenwasserstoffgruppe, insbesondere eine $C_{1-18}$-Alkylengruppe oder eine zweiwertige $C_{1-18}$-Alkylenoxygruppe, beispielsweise eine Gruppe mit 1 bis 8 Kohlenstoffatomen;
$Q^-$ ist ein Halogenidion,
w bedeutet einen statistischen Mittelwert von 2 bis 20 und insbesondere 2 bis 8;
z ist ein statistischer Mittelwert von 20 bis 200 und insbesondere 2 bis 50.

**8.** Emulsion nach einem der Ansprüche 1 bis 7, wobei die organomodifizierten Silicone in Konzentrationen von 0,1 bis 40 %, bezogen auf das Gesamtgewicht der Zusammensetzung, und vorzugsweise in einer Menge von 0,5 bis 20 % enthalten sind.

**9.** Emulsion nach einem der Ansprüche 1 bis 8, wobei die in 3-Stellung unsubstituierte Verbindung vom Typ der Flavyliumsalze der folgenden Formel (1) entspricht:

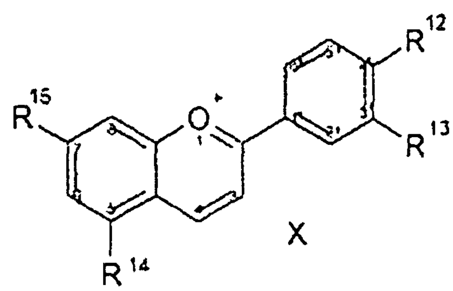

(1)

wobei in der Formel (1) bedeuten:

- $R^{12}$ die OH-Gruppe oder eine geradkettige oder verzweigte, gesättigte oder ungesättigte $C_{1-8}$-Alkoxygruppe,
- $R^{13}$, $R^{14}$ und $R^{15}$, die gleich oder verschieden sind, H oder $R^{12}$, mit der Maßgabe, dass mindestens eine der Gruppen $R^{12}$ bis $R^{15}$ OH bedeutet,
- X- ein organisches oder anorganisches Anion und vorzugsweise ein Halogenid oder ein von einer organischen Säure abgeleitetes Derivat.

**10.** Emulsion nach Anspruch 9, wobei in der Formel (1) $R^{12}$ OH oder $OCH_3$ bedeutet.

**11.** Emulsion nach Anspruch 9 oder 10, wobei die Verbindung der Formel (1) unter den folgenden Chloriden ausgewählt ist:

- 4',5,7-Trihydroxyflavyliumchlorid (oder Apigeninidinchlorid),
- 3',4',7-Trihydroxyflavyliumchlorid,
- 4'-Hydroxyflavyliumchlorid,
- 4',7-Dihydroxyflavyliumchlorid,
- 3',4'-Dihydroxyflavyliumchlorid,
- 3',4'-Dihydroxy-7-methoxyflavyliumchlorid,
- 3',4',5,7-Tetrahydroxyflavyliumchlorid,
- 3',4',5',5,7-Pentahydroxyflavyliumchlorid.

**12.** Emulsion nach Anspruch 11, **dadurch gekennzeichnet, dass** es sich um das 4',5,7-Trihydroxyflavyliumchlorid in reiner Form handelt, das auf synthetischem Wege hergestellt werden kann.

**13.** Emulsion nach Anspruch 11, **dadurch gekennzeichnet, dass** es sich um das 4',5,7-Trihydroxyflavyliumchlorid in Form eines Pflanzenextrakts handelt.

**14.** Emulsion nach Anspruch 13, **dadurch gekennzeichnet, dass** der Pflanzenextrakt ein Pflanzenextrakt ist, der aus den Blättern von Sorghum caudatum; Stängeln, Samen oder Blättern von Sorghum Bicolor; Blütenblättern von Gesneria Fulgens sowie den Arten Blechum Procerum und Sorgho in Kombination mit Colletotrichum Graminicola erhalten wird.

**15.** Emulsion nach Anspruch 13, **dadurch gekennzeichnet, dass** der Pflanzenextrakt ein Extrakt von Sorghum Bicolor ist, der durch eine wässerig-alkoholische saure Extraktion bei einer Extraktionstemperatur von 30 bis 40 °C mit einem Volumenverhältnis Lösungsmittel/Masse der Blätter von Sorghum Bicolor von 10 bis 30 erhältlich ist.

**16.** Emulsion nach Anspruch 15, **dadurch gekennzeichnet, dass** der Extrakt von Sorghum Bicolor 0,05 bis 50 Gew.-% 4',5,7-Trihydroxyflavyliumchlorid enthält.

**17.** Emulsion nach einem der Ansprüche 1 bis 16, wobei die Konzentration der Verbindung vom Typ der Flavyliumsalze im Bereich von 0,0001 bis 10 %, bezogen auf das Gesamtgewicht der Zusammensetzung, liegt.

**18.** Emulsion nach Anspruch 17, wobei die Konzentration der Verbindung vom Typ der Flavyliumsalze im Bereich von 0,001 bis 5 Gew.-%, bezogen auf das Gesamtgewicht der Zusammensetzung, liegt.

**19.** Emulsion nach einem der Ansprüche 1 bis 18, **dadurch gekennzeichnet, dass** sie als Wasser-in-Silicon-Emulsion vorliegt.

**20.** Emulsion nach Anspruch 19, wobei das organomodifizierte Silicon unter den Verbindungen ausgewählt ist, die Alkylenoxidgruppen enthalten, wie sie in Anspruch 2 oder 3 definiert wurden.

**21.** Emulsion nach einem der Ansprüche 1 bis 20, die mindestens 5 Gew.-% eines oder mehrerer polyhydroxylierter Lösungsmittel, bezogen auf das Gewicht der Zusammensetzung, enthält.

**22.** Emulsion nach Anspruch 21, wobei die polyhydroxylierten Lösungsmittel unter den Glykolen und Glykolethern ausgewählt sind.

**23.** Emulsion nach Anspruch 21 oder 22, wobei die polyhydroxylierten Lösungsmittel unter Ethylenglykol, Propylenglykol, Butylenglykol, Dipropylenglykol oder Diethylenglykol und deren Gemischen ausgewählt sind.

**24.** Emulsion nach einem der Ansprüche 21 bis 23, die ein Gemisch von mindestens drei verschiedenen polyhydroxylierten Lösungsmitteln enthält.

**25.** Emulsion nach Anspruch 24, die ein Gemisch enthält, das aus Propylenglykol, Butylenglykol und Dipropylenglykol besteht.

**26.** Verfahren zur kosmetischen Behandlung der Haut, das dazu dient, der Haut eine künstliche Färbung zu geben, die der natürlichen Bräunung nahe kommt, **dadurch gekennzeichnet, dass** es darin besteht, auf die Haut eine

wirksame Menge einer Emulsion nach einem der Ansprüche 1 bis 26 aufzutragen.

**27.** Verwendung mindestens einer Verbindung vom Typ der Flavyliumsalze, die in 3-Stellung nicht substituiert ist und mit mindestens einer Hydroxy- oder Alkoxygruppe substituiert ist und auf synthetischem Wege hergestellt oder aus einem sie enthaltenden Pflanzenextrakt oder einem sie enthaltenden angereicherten Pflanzenextrakt erhalten wird, und mindestens eines organomodifizierten Silicons nach einem der vorhergehenden Ansprüche in kosmetischen und/oder dermatologischen Zusammensetzungen, um der Haut eine künstliche Färbung zu geben, die der natürlichen Bräunung nahe kommt.